# EUROPEAN PATENT APPLICATION

(11) **EP 4 556 459 A1**
(43) Date of publication of application: **21.05.2025**
(21) Application number: 23839602.2
(22) Date of filing: 10.07.2023
(51) Int. Cl.: C07C 213/06, C07C 219/16, C07D 249/08

(54) **COMPOUND SERVING AS PRODUCTION INTERMEDIATE OF AMINO LIPID OR SALT THEREOF, AND METHOD FOR PRODUCING AMINO LIPID COMPOUND USING SAME**

(30) Priority: 11.07.2022 JP 2022111001
(71) Applicant: FUJIFILM Corporation, Tokyo 106-8620 (JP)
(72) Inventor: TANABE, Shintaro, Ashigarakami-gun, Kanagawa 258-8577 (JP); NITABARU, Tatsuya, Ashigarakami-gun, Kanagawa 258-8577 (JP); INOUE, Masaaki, Ashigarakami-gun, Kanagawa 258-8577 (JP); YAMANE, Takehiro, Ashigarakami-gun, Kanagawa 258-8577 (JP); FUKUNAGA, Hirofumi, Ashigarakami-gun, Kanagawa 258-8577 (JP)
(74) Representative: Klunker IP Patentanwälte PartG mbB
(86) International application number: PCT/JP2023/025444
(87) International publication number: WO 2024/014430

(57) **Abstract**

An object of the present invention is to provide a method for manufacturing an aminolipid compound, in which a yield in a carbonate esterification reaction is improved, and an intermediate compound used in the method for manufacturing an aminolipid compound. According to the present invention, there is provided a compound represented by Formula [1] or a salt thereof.

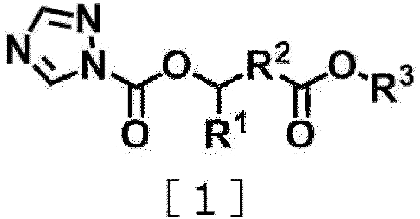

In the formula, R¹ represents a hydrogen atom or a hydrocarbon group having 1 to 12 carbon atoms, R² represents a divalent hydrocarbon group having 2 to 12 carbon atoms, and R³ represents a hydrocarbon group having 6 to 22 carbon atoms.

## Description

### Technical Field

The present invention relates to a compound as a manufacturing intermediate of an aminolipid or a salt thereof, and a method for manufacturing an aminolipid compound using the compound.

### Background Art

Nucleic acid drugs have a clear mechanism of action on diseases, have few side effects, and are expected as next-generation drugs, and nucleic acid drugs are being actively developed. As one technique for delivering a nucleic acid to a cell, a method of encapsulating the nucleic acid in a liposome or a lipid particle and administering the nucleic acid is known. In this technique, as the lipids, lipids having a substituent such as an amino group, which turn into cations at a low pH, are used and appropriate charge is applied to the particles to realize the delivery of nucleic acids.

As the compound to be contained in the lipid particles, Patent Document 1 describes an aminolipid synthesized by condensation of a paranitrophenyl carbonate ester intermediate with an alcohol. In addition, Patent Document 2 describes an aminolipid synthesized by condensation of a 1-imidazolecarboxylate ester intermediate with an alcohol.

On the other hand, Patent Document 3 describes that 1-triazolecarboxylate ester is used as a metal extractant. Patent Document 4 describes that 1-triazolecarboxylate ester is used in a photographic sensitive material. Patent Document 5 describes that 1-triazolecarboxylate ester is used as a synthetic intermediate for an eye disease treatment agent. Patent Document 6 describes a carbonate esterification reaction using 1-triazolecarboxylate ester. Non-Patent Document 1 describes 1-triazolecarboxylate ester as a synthetic intermediate of carbamate which is an amyloid β aggregation inhibitor.

### Prior Art Documents

### Patent Documents

Patent Document 1: WO2019/235635A
Patent Document 2: WO2021/095876A
Patent Document 3: US5254695A
Patent Document 4: JP2003-5323A
Patent Document 5: WO2010/088287A
Patent Document 6: US2015/0133444A

### Non-Patent Documents

Non-Patent Document 1: Molecules 2020, 25, 16, 3610

### SUMMARY OF THE INVENTION

### Object to be solved by the invention

As described above, Patent Document 1 and Patent Document 2 describe that an aminolipid is synthesized by condensation of a paranitrophenyl carbonate ester intermediate or a 1-imidazolecarboxylater ester intermediate with an alcohol, but it is desired to further improve the yield of this synthesis reaction. An object of the present invention is to provide a method for manufacturing an aminolipid compound, in which a yield in a carbonate esterification reaction is improved, and an intermediate compound used in the method for manufacturing an aminolipid compound.

### Means for solving the object

As a result of intensive studies to achieve the above object, the present inventors have found that the yield of an aminolipid is improved by using a 1-triazolecarboxylate ester as an intermediate compound and manufacturing an aminolipid by condensation of the 1-triazolecarboxylate ester with an alcohol. The present invention has been completed based on the above findings. According to the present invention, the following inventions are provided.
<1> A compound represented by Formula [1] or a salt thereof. 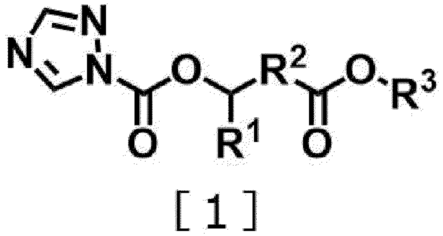 In the formula,
   R¹ represents a hydrogen atom or a hydrocarbon group having 1 to 12 carbon atoms,
   R² represents a divalent hydrocarbon group having 2 to 12 carbon atoms, and
   R³ represents a hydrocarbon group having 6 to 22 carbon atoms.
<2> The compound or a salt thereof according to <1>, in which R¹ represents a hydrocarbon group having 3 to 6 carbon atoms.
<3> The compound or a salt thereof according to <1> or <2>, in which R² represents a divalent hydrocarbon group having 3 to 8 carbon atoms.
<4> The compound or a salt thereof according to any one of <1> to <3>, in which R³ represents a hydrocarbon group having 10 to 14 carbon atoms.
<5> The compound or a salt thereof according to any one of <1> to <4>, in which R³ represents a group represented by -R³¹-C(R³²)R³³, where R³¹ represents a divalent hydrocarbon group having 1 to 5 carbon atoms and R³² and R³³ each independently represent a hydrocarbon group having 1 to 8 carbon atoms.
<6> A compound represented by Formula [1-1], Formula [1-2], Formula [1-3], Formula [1-4], Formula [1-5], Formula [1-6], Formula [1-7], Formula [1-8], or Formula [1-9], or a salt thereof. 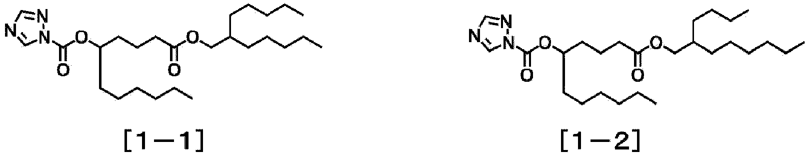 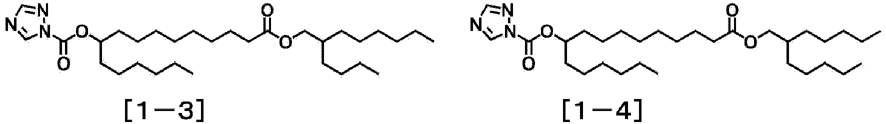 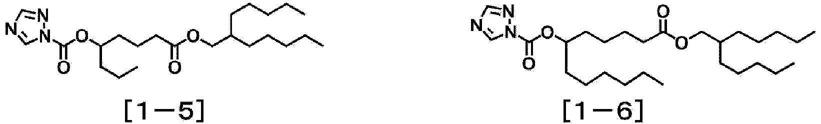 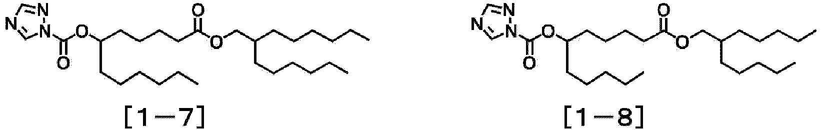 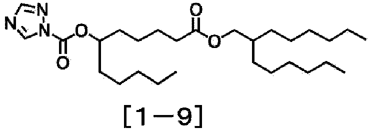
<7> A method for manufacturing a compound represented by Formula [3] or a salt thereof, the method comprising reacting the compound represented by Formula [1] or a salt thereof according to any one of <1> to <6> with a compound represented by Formula [2] or a salt thereof. 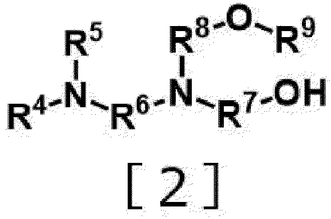 In the formula,
   R⁴ and R⁵ each independently represent a hydrocarbon group having 1 to 3 carbon atoms,
   R⁶ represents a divalent hydrocarbon group having 2 to 4 carbon atoms,
   R⁷ and R⁸ each independently represent a divalent hydrocarbon group having 2 to 4 carbon atoms, and
   R⁹ represents a hydrogen atom or -COR¹⁰, where R¹⁰ represents a hydrocarbon group having 5 to 18 carbon atoms. 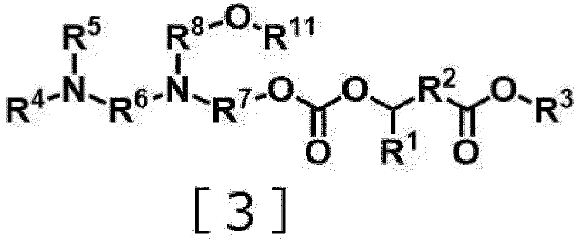 In the formula,
      R¹, R², and R³ have the same definition as R¹, R², and R³ in Formula [1],
      R⁴, R⁵, R⁶, R⁷, and R⁸ have the same definition as R⁴, R⁵, R⁶, R⁷, and R⁸ in Formula [2], and
      R¹¹ represents -COR¹⁰ or 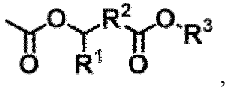 where R¹⁰ has the same definition as R¹⁰ in Formula [2], and R¹, R², and R³ have the same definition as R¹, R², and R³ in Formula [1].
<8> The method according to <7>, in which R⁴ and R⁵ represent a hydrocarbon group having 2 carbon atoms.
<9> The method according to <7> or <8>, in which R⁶ represents a divalent hydrocarbon group having 2 or 3 carbon atoms.
<10> The method according to any one of <7> to <9>, in which R⁷ and R⁸ each independently represent a divalent hydrocarbon group having 2 carbon atoms.
<11> The method according to any one of <7> to <10>, in which R⁹ represents -COR¹⁰, where R¹⁰ represents a hydrocarbon group having 7 to 9 carbon atoms.
<12> The method according to any one of <7> to <11>, in which any one of the following (a), (b), (c), (d), (e), (f), (g), (h), (i), or (j) is applied,
   (a) the compound represented by Formula [1] or a salt thereof is a compound represented by Formula [1-1] or a salt thereof, the compound represented by Formula [2] or a salt thereof is a compound represented by Formula [2-1] or a salt thereof, and the compound represented by Formula [3] or a salt thereof is a compound represented by Formula [3-1] or a salt thereof,
   (b) the compound represented by Formula [1] or a salt thereof is a compound represented by Formula [1-2] or a salt thereof, the compound represented by Formula [2] or a salt thereof is a compound represented by Formula [2-1] or a salt thereof, and the compound represented by Formula [3] or a salt thereof is a compound represented by Formula [3-2] or a salt thereof,
   (c) the compound represented by Formula [1] or a salt thereof is a compound represented by Formula [1-3] or a salt thereof, the compound represented by Formula [2] or a salt thereof is a compound represented by Formula [2-2] or a salt thereof, and the compound represented by Formula [3] or a salt thereof is a compound represented by Formula [3-3] or a salt thereof,
   (d) the compound represented by Formula [1] or a salt thereof is a compound represented by Formula [1-4] or a salt thereof, the compound represented by Formula [2] or a salt thereof is a compound represented by Formula [2-2] or a salt thereof, and the compound represented by Formula [3] or a salt thereof is a compound represented by Formula [3-4] or a salt thereof,
   (e) the compound represented by Formula [1] or a salt thereof is a compound represented by Formula [1-5] or a salt thereof, the compound represented by Formula [2] or a salt thereof is a compound represented by Formula [2-3] or a salt thereof, and the compound represented by Formula [3] or a salt thereof is a compound represented by Formula [3-5] or a salt thereof,
   (f) the compound represented by Formula [1] or a salt thereof is a compound represented by Formula [1-1] or a salt thereof, the compound represented by Formula [2] or a salt thereof is a compound represented by Formula [2-4] or a salt thereof, and the compound represented by Formula [3] or a salt thereof is a compound represented by Formula [3-6] or a salt thereof,
   (g) the compound represented by Formula [1] or a salt thereof is a compound represented by Formula [1-6] or a salt thereof, the compound represented by Formula [2] or a salt thereof is a compound represented by Formula [2-4] or a salt thereof, and the compound represented by Formula [3] or a salt thereof is a compound represented by Formula [3-7] or a salt thereof,
   (h) the compound represented by Formula [1] or a salt thereof is a compound represented by Formula [1-7] or a salt thereof, the compound represented by Formula [2] or a salt thereof is a compound represented by Formula [2-4] or a salt thereof, and the compound represented by Formula [3] or a salt thereof is a compound represented by Formula [3-8] or a salt thereof,
   (i) the compound represented by Formula [1] or a salt thereof is a compound represented by Formula [1-8] or a salt thereof, the compound represented by Formula [2] or a salt thereof is a compound represented by Formula [2-4] or a salt thereof, and the compound represented by Formula [3] or a salt thereof is a compound represented by Formula [3-9] or a salt thereof, or
   (j) the compound represented by Formula [1] or a salt thereof is a compound represented by Formula [1-9] or a salt thereof, the compound represented by Formula [2] or a salt thereof is a compound represented by Formula [2-4] or a salt thereof, and the compound represented by Formula [3] or a salt thereof is a compound represented by Formula [3-10] or a salt thereof.

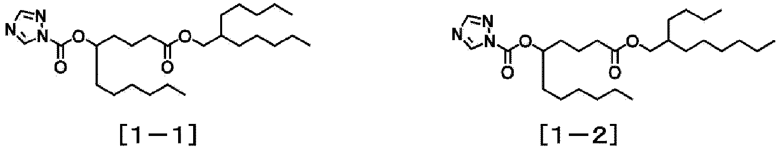

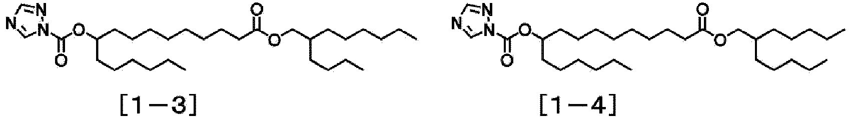

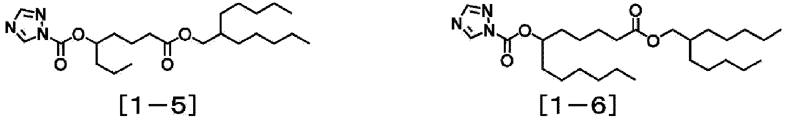

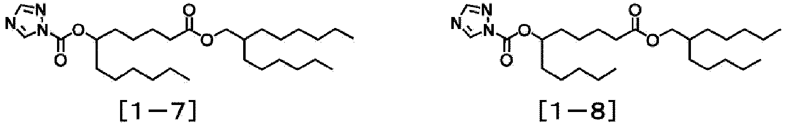

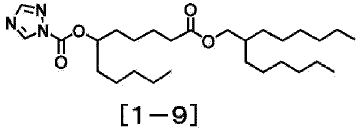

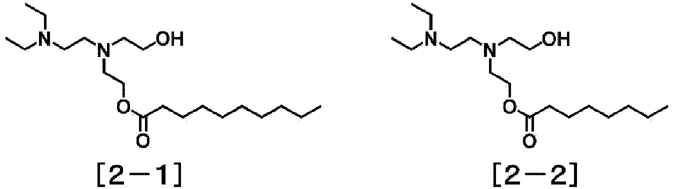

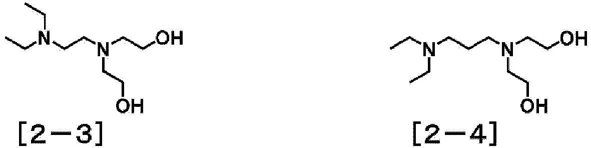

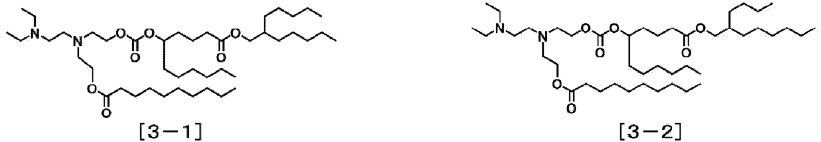

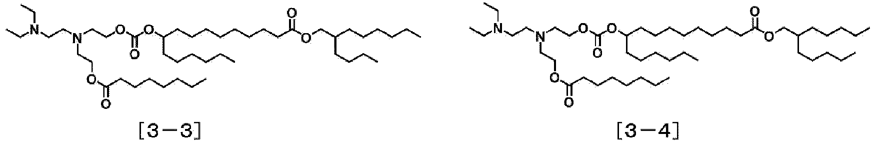

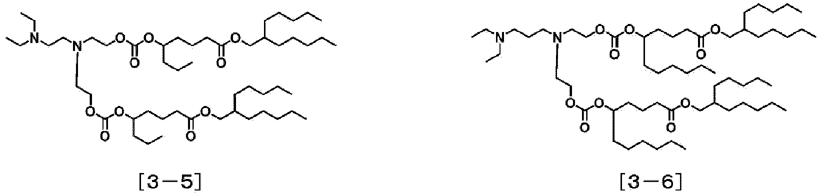

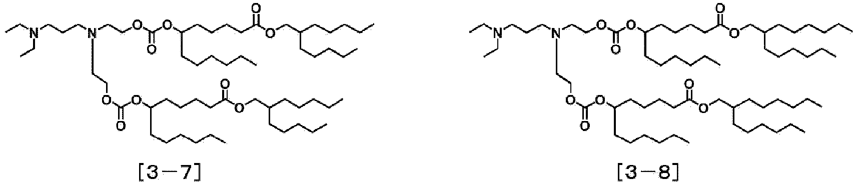

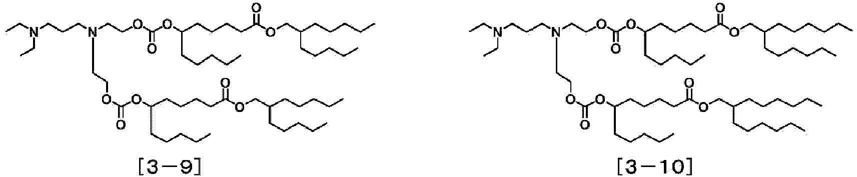

### Effect of the Invention

According to the present invention, the yield in the carbonate esterification reaction can be improved, and an aminolipid compound can be manufactured with a high yield.

### BRIEF DESCRIPTION OF THE DRAWINGS

[Fig. 1] Fig. 1 shows the results of analyzing the change of a compound [2-1] under the conditions of Comparative Example 1 by silica gel thin layer chromatography.
[Fig. 2] Fig. 2 shows the results of analysis by silica gel thin layer chromatography in an imidazole addition experiment.

### EMBODIMENTS FOR CARRYING OUT THE INVENTION

Hereinafter, the present invention will be specifically described.

In the present specification, "to" shows a range including numerical values described before and after "to" as a minimum value and a maximum value respectively.

### <Intermediate for manufacturing aminolipid or salt thereof>

The present invention relates to a compound represented by Formula [1] or a salt thereof. The compound represented by Formula [1] or a salt thereof can be used as an intermediate for manufacturing an aminolipid or a salt thereof. 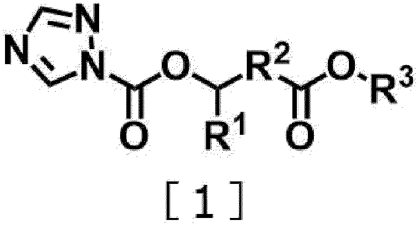 In the formula,
R¹ represents a hydrogen atom or a hydrocarbon group having 1 to 12 carbon atoms,
R² represents a divalent hydrocarbon group having 2 to 12 carbon atoms, and
R³ represents a hydrocarbon group having 6 to 22 carbon atoms.

R¹ preferably represents a hydrocarbon group having 2 to 10 carbon atoms, more preferably represents a hydrocarbon group having 2 to 8 carbon atoms, and still more preferably represents a hydrocarbon group having 3 to 6 carbon atoms, and for example, represents a hydrocarbon group having 3 or 6 carbon atoms.

R² preferably represents a divalent hydrocarbon group having 2 to 10 carbon atoms, and more preferably represents a divalent hydrocarbon group having 3 to 8 carbon atoms, and for example, represents a divalent hydrocarbon group having 3 or 8 carbon atoms.

R³ preferably represents a hydrocarbon group having 8 to 18 carbon atoms, more preferably represents a hydrocarbon group having 8 to 16 carbon atoms, and still more preferably represents a hydrocarbon group having 10 to 14 carbon atoms, and examples thereof include a hydrocarbon group having 12 carbon atoms.

R³ preferably represents a group represented by -R³¹-C(R³²)R³³, where R³¹ represents a divalent hydrocarbon group having 1 to 5 carbon atoms, and R³² and R³³ each independently represent a hydrocarbon group having 1 to 8 carbon atoms.

R³¹ preferably represents a divalent hydrocarbon group having 1 to 3 carbon atoms, more preferably represents a divalent hydrocarbon group having 1 or 2 carbon atoms, and still more preferably represents a divalent hydrocarbon group having 1 carbon atom.

R³² and R³³ each independently more preferably represent a hydrocarbon group having 2 to 7 carbon atoms and still more preferably represent a hydrocarbon group having 4 to 6 carbon atoms.

As the hydrocarbon group having 1 to 12 carbon atoms represented by R¹, an alkyl group having 1 to 12 carbon atoms, an alkenyl group having 2 to 12 carbon atoms, or an alkynyl group having 2 to 12 carbon atoms is preferable, and an alkyl group having 1 to 12 carbon atoms is more preferable. The hydrocarbon group having 1 to 12 carbon atoms may be linear or branched, or may be chain-like or cyclic. Specific examples of the hydrocarbon group having 1 to 12 carbon atoms include a methyl group, an ethyl group, a propyl group, an isopropyl group, a cyclopropyl group, a butyl group, an isobutyl group, a tert-butyl group, a cyclobutyl group, a pentyl group, a cyclopentyl group, a hexyl group, a cyclohexyl group, a heptyl group, an octyl group, a nonyl group, a decyl group, an undecyl group, and a dodecyl group.

The divalent hydrocarbon group having 2 to 12 carbon atoms represented by R² is preferably an alkylene group having 2 to 12 carbon atoms or an alkenylene group having 2 to 12 carbon atoms. The divalent hydrocarbon group having 2 to 12 carbon atoms may be linear or branched, or may be chain-like or cyclic. Specific examples of the divalent hydrocarbon group having 2 to 12 carbon atoms include a methylene group, an ethylene group, a trimethylene group, a tetramethylene group, a pentamethylene group, a hexamethylene group, a heptamethylene group, an octamethylene group, a nonamethylene group, a decamethylene group, an undecamethylene group, and a dodecamethylene group.

As the hydrocarbon group having 6 to 22 carbon atoms represented by R³, an alkyl group having 6 to 22 carbon atoms, an alkenyl group having 6 to 22 carbon atoms, or an alkynyl group having 6 to 22 carbon atoms is preferable, and an alkyl group having 6 to 22 carbon atoms is more preferable. The hydrocarbon group having 6 to 22 carbon atoms may be linear or branched, or may be chain-like or cyclic. Specific examples of the hydrocarbon group having 6 to 22 carbon atoms include a hexyl group, a heptyl group, an octyl group, a nonyl group, a decyl group, an undecyl group, a dodecyl group, a tridecyl group, a trimethyldodecyl group (preferably a 3,7,11-trimethyldodecyl group), a tetradecyl group, a pentadecyl group, a hexadecyl group, a tetramethylhexadecyl group (preferably a 3,7,11,15-tetramethylhexadecyl group), a heptadecyl group, an octadecyl group, a nonadecyl group, and an icosyl group.

The particularly preferred compound as the compound represented by Formula [1] or a salt thereof is a compound represented by Formula [1-1], Formula [1-2], Formula [1-3], Formula [1-4], Formula [1-5], Formula [1-6], Formula [1-7], Formula [1-8], or Formula [1-9], or a salt thereof.

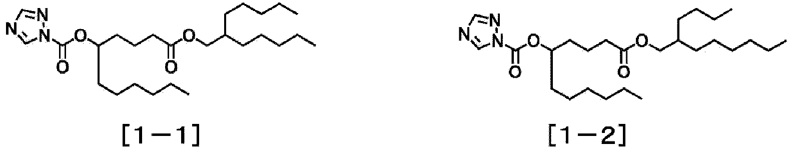

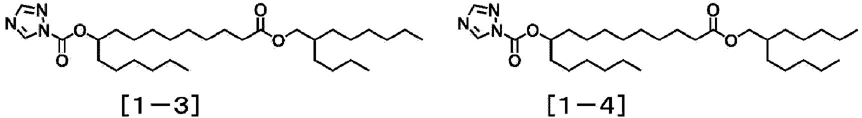

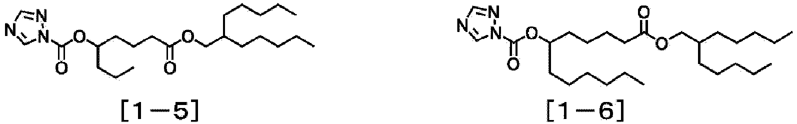

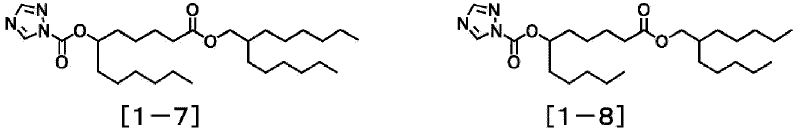

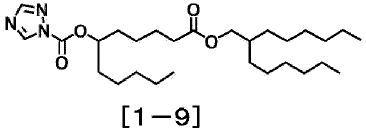

The compound represented by Formula (1) may form a salt.

Examples of the salt in a basic group include salts with mineral acids such as hydrochloric acid, hydrobromic acid, nitric acid, and sulfuric acid; salts with organic carboxylic acids such as formic acid, acetic acid, citric acid, oxalic acid, fumaric acid, maleic acid, succinic acid, malic acid, tartaric acid, aspartic acid, trichloroacetic acid, and trifluoroacetic acid; and salts with sulfonic acids such as methanesulfonic acid, benzenesulfonic acid, p-toluenesulfonic acid, mesitylenesulfonic acid, and naphthalenesulfonic acid.

Among the above salts, preferred examples of the salt include pharmacologically acceptable salts.

The compound represented by Formula [1] can be manufactured according to the method described in Examples which will be described later.

Specifically, the compound represented by Formula [4] and 1,1'-carbonyldi(1,2,4-triazole) may be reacted with each other in the presence of an appropriate solvent.

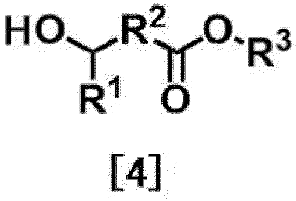

In the formula, the definitions of R¹, R², and R³ are the same as the definitions of R¹, R², and R³ in Formula [1].

The solvent used in this reaction is not particularly limited as long as the solvent does not affect the reaction. Examples of the solvent include ethers, acetonitrile, halogenated hydrocarbons, amides, and aromatic hydrocarbons. These solvents may be used by being mixed together. The preferred solvent is ethers (for example, tetrahydrofuran) or acetonitrile.

As the reaction reagent, amines such as triethylamine, 1,8-diazabicyclo[5.4.0]-7-undecene, or the like can be used.

The used amount of the solvent is not particularly limited, but may be 1 to 500 times (v/w) the amount of the compound represented by Formula [4].

The used amount of the compound represented by Formula [4] is not particularly limited, but may be 0.1 to 10 times the molar amount of 1,1'-carbonyldi(1,2,4-triazole).

This reaction may be carried out at 0°C to 100°C, preferably 10°C to 80°C, for 10 minutes to 48 hours, preferably 1 hour to 24 hours.

### <Method for manufacturing aminolipid compound>

The present invention further relates to a method for manufacturing a compound represented by Formula [3] or a salt thereof, the method including reacting the compound represented by Formula [1] or a salt thereof with the compound represented by Formula [2] or a salt thereof.

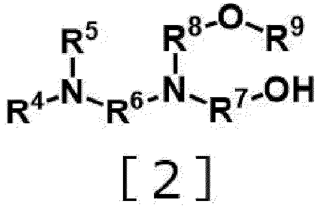

In the formula,
R⁴ and R*⁵* each independently represent a hydrocarbon group having 1 to 3 carbon atoms,
R⁶ represents a divalent hydrocarbon group having 2 to 4 carbon atoms,
R⁷ and R⁸ each independently represent a divalent hydrocarbon group having 2 to 4 carbon atoms, and
R⁹ represents a hydrogen atom or -COR¹⁰, where R¹⁰ represents a hydrocarbon group having 5 to 18 carbon atoms.

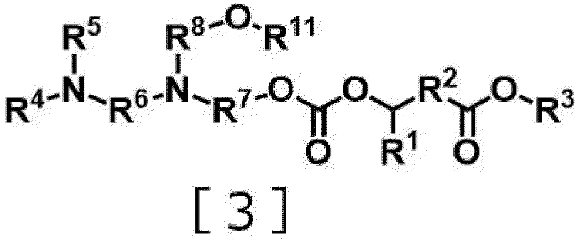

In the formula,
R¹, R², and R³ have the same definition as R¹, R², and R³ in Formula [1],
R⁴, R⁵, R⁶, R⁷, and R⁸ have the same definition as R⁴, R⁵, R⁶, R⁷, and R⁸ in Formula [2], and
R¹¹ represents -COR¹⁰ or 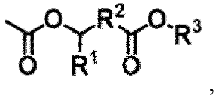 where R¹⁰ has the same definition as R¹⁰ in Formula [2], and R¹, R², and R³ have the same definition as R¹, R², and R³ in Formula [1].

In a case where a paranitrophenyl carbonate ester is used as an intermediate, it is considered that addition of N,N-dimethylaminopyridine (DMAP) is required for the progress of the carbonate esterification reaction, but the separation from DMAP by column purification in the purification step is difficult, which results in a decrease in an isolation yield. In addition, in a case where 1-imidazolecarboxylate ester is used as an intermediate, it is considered that, in the carbonate esterification reaction, imidazole that is eliminated as the reaction proceeds causes the disproportionation of the ester, which generates a plurality of by-products and thus the yield is decreased. On the other hand, in the present invention, by using the 1-triazolecarboxylate ester represented by Formula [1], the yield is improved as compared with the method using the paranitrophenyl carbonate ester or the 1-imidazolecarboxylate ester. In the method of the present invention, it is presumed that since the additive is not required, the decrease in the yield in the purification step is not observed, and since the reactivity of the triazole eliminated as the reaction proceeds is lower than that of imidazole, the disproportionation of the ester does not occur.

R⁴ and R⁵ each preferably represent a hydrocarbon group having 2 carbon atoms. R⁶ preferably represents a divalent hydrocarbon group having 2 or 3 carbon atoms.

R⁷ and R⁸ each preferably represent a divalent hydrocarbon group having 2 carbon atoms.

R⁹ preferably represents -COR¹⁰, where R¹⁰ preferably represents a hydrocarbon group having 7 to 9 carbon atoms.

The hydrocarbon group having 1 to 3 carbon atoms represented by R⁴ and R⁵ may be any one of an alkyl group, an alkenyl group, or an alkynyl group, but an alkyl group is preferable. Specific examples of the hydrocarbon group having 1 to 3 carbon atoms represented by R⁴ and R⁵ include a methyl group, an ethyl group, and a propyl group.

The divalent hydrocarbon group having 2 to 4 carbon atoms represented by R⁶ is preferably an alkylene group or an alkenylene group, and more preferably an alkylene group. Specific examples of the divalent hydrocarbon group having 2 to 4 carbon atoms include an ethylene group, a trimethylene group, and a tetramethylene group.

The divalent hydrocarbon group having 2 to 4 carbon atoms represented by R⁷ and R⁸ is preferably an alkylene group or an alkenylene group, and more preferably an alkylene group. Specific examples of the divalent hydrocarbon group having 2 to 4 carbon atoms include an ethylene group, a trimethylene group, and a tetramethylene group.

As the hydrocarbon group having 5 to 18 carbon atoms represented by R¹⁰, an alkyl group having 5 to 18 carbon atoms, an alkenyl group having 5 to 18 carbon atoms, or an alkynyl group having 5 to 18 carbon atoms is preferable, and an alkyl group having 5 to 18 carbon atoms is more preferable. The hydrocarbon group having 5 to 18 carbon atoms may be linear or branched, or may be chain-like or cyclic. Examples of the hydrocarbon group having 5 to 18 carbon atoms include a hexyl group, a heptyl group, an octyl group, a nonyl group, a decyl group, an undecyl group, a dodecyl group, a tridecyl group, a trimethyldodecyl group (preferably a 3,7,11-trimethyldodecyl group), a tetradecyl group, a pentadecyl group, a hexadecyl group, a heptadecyl group, an octadecyl group, and the like.

In the method for manufacturing an aminolipid compound according to the embodiment of the present invention, any one of the following (a), (b), (c), (d), (e), or (f) is particularly preferable.
(a) the compound represented by Formula [1] or a salt thereof is a compound represented by Formula [1-1] or a salt thereof, the compound represented by Formula [2] or a salt thereof is a compound represented by Formula [2-1] or a salt thereof, and the compound represented by Formula [3] or a salt thereof is a compound represented by Formula [3-1] or a salt thereof,
(b) the compound represented by Formula [1] or a salt thereof is a compound represented by Formula [1-2] or a salt thereof, the compound represented by Formula [2] or a salt thereof is a compound represented by Formula [2-1] or a salt thereof, and the compound represented by Formula [3] or a salt thereof is a compound represented by Formula [3-2] or a salt thereof,
(c) the compound represented by Formula [1] or a salt thereof is a compound represented by Formula [1-3] or a salt thereof, the compound represented by Formula [2] or a salt thereof is a compound represented by Formula [2-2] or a salt thereof, and the compound represented by Formula [3] or a salt thereof is a compound represented by Formula [3-3] or a salt thereof, or
(d) the compound represented by Formula [1] or a salt thereof is a compound represented by Formula [1-4] or a salt thereof, the compound represented by Formula [2] or a salt thereof is a compound represented by Formula [2-2] or a salt thereof, and the compound represented by Formula [3] or a salt thereof is a compound represented by Formula [3-4] or a salt thereof.
(e) The compound represented by Formula [1] or a salt thereof is a compound represented by Formula [1-5] or a salt thereof, the compound represented by Formula [2] or a salt thereof is a compound represented by Formula [2-3] or a salt thereof, and the compound represented by Formula [3] or a salt thereof is a compound represented by Formula [3-5] or a salt thereof.
(f) The compound represented by Formula [1] or a salt thereof is a compound represented by Formula [1-1] or a salt thereof, the compound represented by Formula [2] or a salt thereof is a compound represented by Formula [2-4] or a salt thereof, and the compound represented by Formula [3] or a salt thereof is a compound represented by Formula [3-6] or a salt thereof.
(g) The compound represented by Formula [1] or a salt thereof is a compound represented by Formula [1-6] or a salt thereof, the compound represented by Formula [2] or a salt thereof is a compound represented by Formula [2-4] or a salt thereof, and the compound represented by Formula [3] or a salt thereof is a compound represented by Formula [3-7] or a salt thereof,
(h) the compound represented by Formula [1] or a salt thereof is a compound represented by Formula [1-7] or a salt thereof, the compound represented by Formula [2] or a salt thereof is a compound represented by Formula [2-4] or a salt thereof, and the compound represented by Formula [3] or a salt thereof is a compound represented by Formula [3-8] or a salt thereof,
(i) the compound represented by Formula [1] or a salt thereof is a compound represented by Formula [1-8] or a salt thereof, the compound represented by Formula [2] or a salt thereof is a compound represented by Formula [2-4] or a salt thereof, and the compound represented by Formula [3] or a salt thereof is a compound represented by Formula [3-9] or a salt thereof, or
(j) the compound represented by Formula [1] or a salt thereof is a compound represented by Formula [1-9] or a salt thereof, the compound represented by Formula [2] or a salt thereof is a compound represented by Formula [2-4] or a salt thereof, and the compound represented by Formula [3] or a salt thereof is a compound represented by Formula [3-10] or a salt thereof.

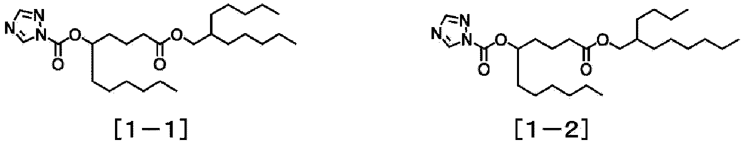

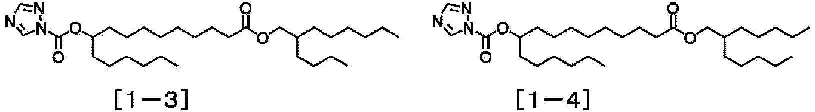

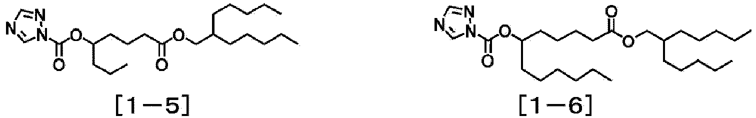

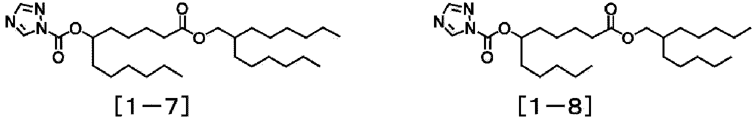

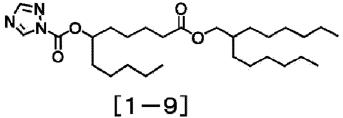

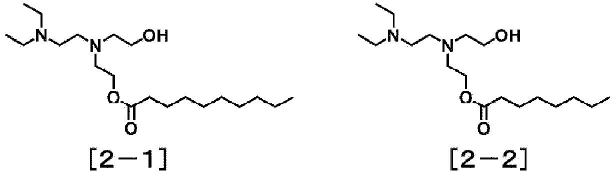

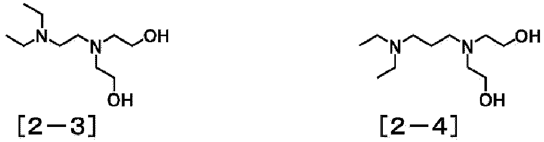

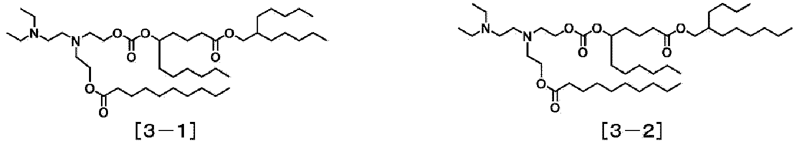

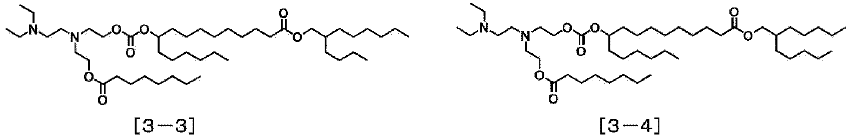

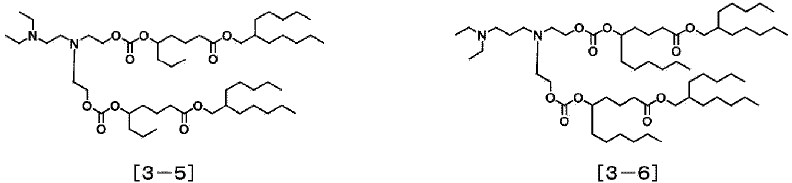

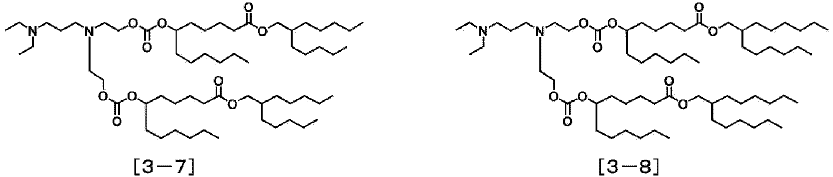

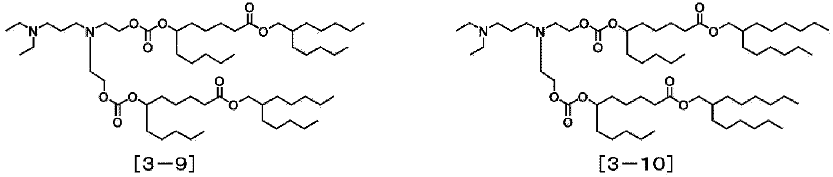

The reaction of the compound represented by Formula [1] or a salt thereof with the compound represented by Formula [2] or a salt thereof can be carried out according to the method described in Examples which will be described later.

Specifically, the compound represented by Formula [1] or a salt thereof and the compound represented by Formula [2] or a salt thereof may be reacted with each other in the presence of an appropriate solvent and an appropriate base.

The solvent used in this reaction is not particularly limited as long as the solvent does not affect the reaction. Examples of the solvent include ethers, acetonitrile, halogenated hydrocarbons, amides, and aromatic hydrocarbons. These solvents may be used by being mixed together. The preferred solvent is ethers (for example, tetrahydrofuran) or acetonitrile.

As the base, an organic base is preferable, and amines such as triethylamine or 1,8-diazabicyclo[5.4.0]-7-undecene, or the like can be used.

The used amount of the solvent is not particularly limited, but may be 1 to 500 times (v/w) the amount of the compound represented by Formula [1].

The used amount of the compound represented by Formula [1] is not particularly limited, but may be 0.1 to 10 times the molar amount of the compound represented by Formula [2].

This reaction may be carried out at 0°C to 100°C, preferably 10°C to 80°C, for 10 minutes to 48 hours, preferably 1 hour to 36 hours.

### <Use application of aminolipid>

The aminolipid or a salt thereof, which is manufactured by the manufacturing method of the present invention, can be used for preparing lipid particles. In a case of preparing the lipid particles, in addition to the aminolipid or a salt thereof, at least one type of lipid selected from the group consisting of a sterol, a neutral lipid, and a lipid having a nonionic hydrophilic polymer chain can be used. The lipid particles can further contain a nucleic acid.

In the lipid particles, the blending amount of the aminolipid with respect to the total amount of lipids is preferably 20 mol% to 80 mol%, more preferably 35 mol% to 70 mol%, and still more preferably 40 mol% to 65 mol%.

The sterol is not particularly limited, and examples thereof can include cholesterol, phytosterol (sitosterol, stigmasterol, fucosterol, spinasterol, brassicasterol, and the like), ergosterol, cholestanone, cholestenone, coprostanol, cholesteryl-2'-hydroxyethyl ether, and cholesteryl-4'-hydroxybutyl ether. Among these, cholesterol is preferable. In the present invention, the blending amount of the sterol with respect to the total amount of lipids is preferably 10 mol% to 60 mol%, more preferably 20 mol% to 55 mol%, and still more preferably 25 mol% to 50 mol%.

The neutral lipid is not particularly limited, and examples thereof include phosphatidylcholine, phosphatidylethanolamine, sphingomyelin, ceramide, and the like. Among these, phosphatidylcholine and phosphatidylethanolamine are preferable. The neutral lipid may be a single neutral lipid or a combination of a plurality of different neutral lipids. In the lipid particles, the blending amount of the neutral lipid with respect to the total amount of the constituent lipid components is preferably 3 mol% or more and 55 mol% or less.

Examples of the nonionic hydrophilic polymer in the lipid having a nonionic hydrophilic polymer chain are not particularly limited, and examples thereof include a nonionic vinyl-based polymer, a nonionic polyamino acid, a nonionic polyester, a nonionic polyether, a nonionic natural polymer, a nonionic modified natural polymer, and a block polymer or a graft copolymer having two or more kinds of these polymers as constitutional units. Among these nonionic hydrophilic polymers, a nonionic polyether, a nonionic polyester, a nonionic polyamino acid, or a nonionic synthetic polypeptide is preferable, a nonionic polyether or a nonionic polyester is more preferable, a nonionic polyether or a nonionic monoalkoxy polyether is still more preferable, and polyethylene glycol (hereinafter, polyethylene glycol will be also called PEG) is particularly preferable.

The lipid having a nonionic hydrophilic polymer is not particularly limited, and examples thereof include PEG-modified phosphoethanolamine, a diacylglycerol PEG derivative, a dialkylglycerol PEG derivative, a cholesterol PEG derivative, a ceramide PEG derivative, and the like. Among these, diacylglycerol PEG is preferable.

The weight-average molecular weight of the PEG chain of the nonionic hydrophilic polymer derivative is preferably 500 to 5,000 and more preferably 750 to 3,000.

The nonionic hydrophilic polymer chain may be branched or may have a substituent such as a hydroxymethyl group.

In the lipid particles, the blending amount of the lipid having a nonionic hydrophilic polymer chain with respect to the total amount of lipids is preferably 0.25 mol% to 12 mol%, more preferably 0.5 mol% to 6 mol%, and still more preferably 1 mol% to 3 mol%.

The lipid particles may contain a nucleic acid. Examples of the nucleic acid include a plasmid, single-stranded DNA, double-stranded DNA, small interfering RNA (siRNA), micro RNA (miRNA), mRNA, an antisense oligonucleotide, ribozyme, an aptamer, saRNA, sgRNA, and the like. The lipid particles may contain any of these. In addition, the lipid composition may contain a modified nucleic acid. As the nucleic acid, RNA is particularly preferable, and RNA having a number of bases of 5 to 20,000 is preferable.

In the lipid particles, the mass ratio of the lipid to the nucleic acid is preferably 2 to 1,000, more preferably 3 to 500, even more preferably 5 to 200, and particularly preferably 5 to 100.

The method for manufacturing the lipid particles is not limited. For example, the lipid particles can be manufactured by a method in which all of the constituent components of the lipid particles or some of oil-soluble components of the lipid particles are dissolved in an organic solvent or the like such that an oil phase is formed, water-soluble components of the lipid particles are dissolved in water such that a water phase is formed, and the oil phase and the water phase are mixed together. A micromixer may be used for mixing, or an emulsification using an emulsifying machine such as a homogenizer, an ultrasonic emulsifying machine, a high-pressure injection emulsifying machine, or the like may be performed.

Alternatively, the lipid particles can also be manufactured by subjecting a solution containing a lipid to evaporation to dryness using an evaporator or the like under reduced pressure or to spray drying using a spray drier or the like, to prepare a dried mixture containing the lipid, adding this mixture to an aqueous solvent, and further emulsifying using the above-described emulsifying machine or the like.

Examples of the method for manufacturing lipid particles containing a nucleic acid include a method including
a step (a) of dissolving constituent components of lipid particles containing the compound according to the embodiment of the present invention in an organic solvent to obtain an oil phase,
a step (b) of mixing the oil phase obtained in the step (a) with a water phase containing a nucleic acid,
a step (c) of diluting the mixed solution containing the oil phase and the water phase obtained in the step (b) to obtain a dispersion liquid of nucleic acid lipid particles, and
a step (d) of removing the organic solvent from the dispersion liquid of the nucleic acid lipid particles obtained in the step (c).

Lipid particles mean particles composed of a lipid, and include a composition having any structure selected from a lipid aggregate in which lipids are aggregated, a micelle, or a liposome. The structure of the lipid particles is not limited to these as long as the lipid particles are a composition containing a lipid. The liposome has a lipid bilayer structure and has a water phase in the inside, and includes a liposome which has a single bilayer membrane, and a multiphase liposome which has multiple layers stacked together. The present invention may include any of these liposomes.

The form of the lipid particles can be checked by electron microscopy, structural analysis using X-rays, or the like. For example, by a method using Cryo transmission electron microscopy (CryoTEM method), it is possible to check, for example, whether or not a lipid particle is, such as a liposome, a bimolecular lipid membrane structure (lamella structure) and a structure having an inner water layer, and whether or not a lipid particle has a structure having an inner core with a high electron density and packed with constituent components including a lipid. The X-ray small angle scattering (SAXS) measurement also makes it possible to check whether or not a lipid particle has a bimolecular lipid membrane structure (lamella structure).

As one example for utilizing the lipid particles, a nucleic acid (for example, a gene) can be introduced into a cell by introducing the lipid particles containing the nucleic acid into the cell. In addition, in a case where the lipid particles in the present invention contain a nucleic acid for a pharmaceutical use, the lipid particles can be administered to a living body as a nucleic acid drug.

The lipid particles can retain a nucleic acid at a high encapsulation rate. Therefore, the lipid particles are extremely useful as a nucleic acid delivery carrier. According to the nucleic acid delivery carrier using the present invention, for example, by mixing the obtained lipid particles with a nucleic acid or the like and performing transfection in vitro or in vivo, the nucleic acid and the like can be introduced into cells. The nucleic acid delivery carrier is also useful as a nucleic acid delivery carrier in nucleic acid drugs. That is, the lipid particles are useful as a composition for in vitro or in vivo (preferably in vivo) delivery of a nucleic acid.

Next, the present invention will be described using Examples, but the present invention is not limited thereto.

### Examples

Unless otherwise specified, in purification by silica gel column chromatography, the automatic purification device ISOLERA (Biotage AB, Inc.), the medium pressure preparative purification device Purif-espoir-2 (Shoko Science Co., Ltd.), the medium pressure liquid chromatograph YFLC W-prep 2XY (Yamazen Corporation), or a glass open column chromatographic tube was used.

Unless otherwise specified, as a carrier in silica gel column chromatography, Chromatorex Q-Pack SI 50 (FUJI SILYSIA CHEMICAL LTD.), or high flash column WO01, WO02, WO03, WO04, or WO05 (Yamazen Corporation), Wakosil C-200 (FUJIFILM Wako Pure Chemical Corporation), or CHROMATOREX PSQ 100B (FUJI SILYSIA CHEMICAL LTD.) was used.

For NH silica gel, Chromatorex Q-Pack NH 60 (FUJI SILYSIA CHEMICAL LTD.) or CHROMATOREX NH-DM1020 (FUJI SILYSIA CHEMICAL LTD.) was used.

NMR spectra were measured using AVNEO400 (manufactured by Bruker Corporation) and using tetramethylsilane as an internal standard, and all δ values were shown in ppm.

MS spectra were measured using an ACQUITY SQD LC/MS System (manufactured by Waters Corporation).

### [Example 1]

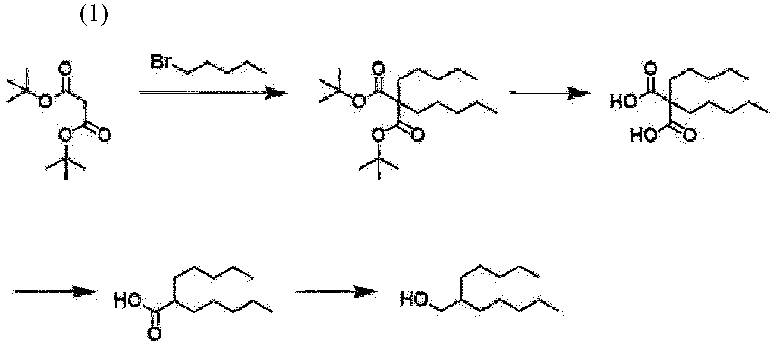

Di-tert-butyl malonate (25.00 g) was added to a mixture of dimethyl sulfoxide (125 mL), toluene (105 mL), and powdered potassium hydroxide (16.6 g) under ice cooling, the mixture was stirred at the same temperature for 10 minutes, then 1-bromopentane (41.9 g) was added thereto, and the mixture was stirred at room temperature for 5 hours. 7% hydrochloric acid (160 g) was added dropwise to the reaction mixture under ice cooling, and then the organic layer was separated and washed with water (50 g) and a 20% aqueous sodium chloride solution (60 g). After drying over anhydrous sodium sulfate (50 g), the solvent was distilled off under reduced pressure to obtain di-tert-butyl 2,2-dipentylmalonate (54.8 g) as a light yellow oily substance.

¹H-NMR(CDCl₃) δ: 1.80-1.74 (4H,m), 1.44 (18H,s), 1.36-1.23 (8H,m), 1.18-1.09 (4H,m), 0.87 (6H,t,J = 7.0 Hz).

Water (5.4 mL), toluene (54 mL), and trifluoroacetic acid (108 mL) were added to the obtained di-tert-butyl 2,2-dipentylmalonate (54.4 g), and the mixture was stirred at room temperature for 2 hours. After distilling off the solvent of the reaction mixture under reduced pressure, hexane (88 mL) was added thereto, and the mixture was stirred under ice cooling for 30 minutes. The solid matter was collected by filtration and then washed with hexane to obtain 2,2-dipentylmalonic acid (15.4 g) as a white solid.

¹H-NMR(CDCl₃) δ: 1.98-1.91 (4H, m), 1.35-1.22 (12H, m), 0.87 (6H, t, J = 7.0 Hz).

2,2-Dipentylmalonic acid (18.0 g) was stirred at 165°C for 3 hours in a solvent-free state, and then cooled to room temperature to obtain 2-pentylheptanoic acid (14.8 g) as a light yellow oily substance.

¹H-NMR(CDCl₃) δ: 2.39-2.31 (1H, m), 1.67-1.56 (2H, m), 1.52-1.42 (2H, m), 1.36-1.22 (12H, m), 0.90-0.85 (6H, m).

A tetrahydrofuran solution (200 mL) of 2-pentylheptanoic acid (98.5 g) was added dropwise to a mixture of lithium aluminum hydride (39.2 g) and tetrahydrofuran (800 mL) under ice cooling, and the mixture was stirred at room temperature for 1 hour. Sodium sulfate decahydrate (200 g) was added to the reaction mixture under ice cooling, and the mixture was stirred at room temperature for 5 hours. After filtering off the insoluble matter, the solvent was distilled off under reduced pressure, and the obtained residue was purified by silica gel column chromatography (ethyl acetate-hexane) to obtain 2-pentylheptan-1-ol (89.1 g) as a light yellow oily substance.

¹H-NMR(CDCl₃) δ: 3.57-3.51 (2H, m), 1.50-1.20 (17H, m), 0.88 (6H, t, J = 6.6 Hz).

(2)

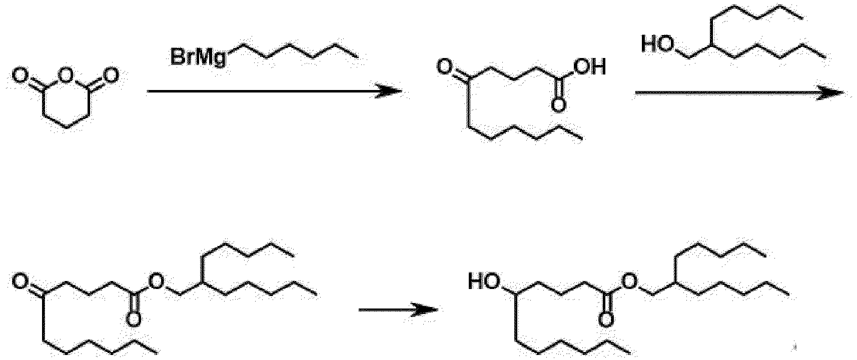

A mixture of a 20% hexylmagnesium bromide-tetrahydrofuran solution (510 g) and tetrahydrofuran (260 mL) was added dropwise to a tetrahydrofuran solution (255 mL) of glutaric anhydride (68.1 g) under ice cooling, the mixture was stirred at the same temperature for 30 minutes, and then 7% hydrochloric acid (540 mL) was added dropwise thereto. Hexane (480 mL) was added thereto, the mixture was stirred at room temperature for 5 minutes, the organic layer was separated and washed with water (240 mL), then a mixture of potassium hydroxide (74.9 g), methanol (375 mL), and water (780 mL) was added thereto, and the aqueous layer was separated. After washing with methyl tert-butyl ether (370 mL), 30% hydrochloric acid (190 mL) was added under ice cooling, ethyl acetate (375 mL) was added thereto, and the organic layer was separated and washed with water (140 mL). The organic layer was dried over anhydrous sodium sulfate (24.2 g), and then the solvent was distilled off under reduced pressure. Methanol (560 mL) was added to the obtained brown solid (67.7 g), water (1,700 mL) was added dropwise thereto, and the mixture was stirred under ice cooling for 10 minutes. The solid matter was collected by filtration and washed three times with water (190 mL) to obtain a light brown solid (48.9 g). Methyl tert-butyl ether (90 mL) was added to the obtained light brown solid (44.9 g), the mixture was stirred at 40°C, hexane (360 mL) was added thereto, and the mixture was stirred under ice cooling. The solid matter was collected by filtration and washed three times with hexane (180 mL) to obtain 5-oxoundecanoic acid (31.1 g) as a white solid.

¹H-NMR(CDCl₃) δ: 2.50 (2H, t, J = 7.2 Hz), 2.40 (4H, t, J = 7.2 Hz), 2.02-1.80 (2H, m), 1.63-1.48 (2H, m), 1.37-1.20 (6H, m), 0.88 (3H, t, J = 6.6 Hz).

p-Toluenesulfonic acid monohydrate (1.7 g) was added to a mixture of 5-oxoundecanoic acid (40.8 g), 2-pentylheptane-1-ol (33.0 g), and toluene (165 mL), and the mixture was stirred under heating and reflux for 4 hours. The reaction mixture was cooled to room temperature, then hexane (150 mL) and water (300 mL) were added thereto, and the organic layer was separated. The organic layer was washed twice with water (300 mL), and then washed with a saturated aqueous sodium chloride solution (300 mL). After drying over anhydrous sodium sulfate (50 g), the solvent was distilled off under reduced pressure to obtain 2-pentylheptyl 5-oxoundecanoate (75.5 g) as a light yellow oily substance.

¹H-NMR(CDCl₃) δ: 3.97 (2H, d, J = 5.1 Hz), 2.47 (2H, t, J = 7.2 Hz), 2.39 (2H, t, J = 7.2 Hz), 2.33 (2H, t, J = 7.2 Hz), 1.95-1.83 (2H, m), 1.66-1.49 (3H, m), 1.36-1.20 (22H, m), 0.92-0.82 (9H, m).

Toluene (375 mL) was added to the obtained 2-pentylheptyl 5-oxoundecanoate (75.5 g), sodium borohydride (5.8 g) was added thereto under ice cooling, methanol (75 mL) was added dropwise thereto at the same temperature, and the mixture was stirred for 1 hour. Hexane (500 mL) and 1 mol/L hydrochloric acid (250 mL) were added to the reaction mixture at the same temperature, then water (200 mL) was added thereto, and the organic layer was separated. The organic layer was washed twice with water (500 mL) and once with a saturated aqueous sodium chloride solution (300 mL), and dried over anhydrous sodium sulfate (50 g), and the solvent was distilled off under reduced pressure. The obtained residue was purified by silica gel column chromatography (ethyl acetate-hexane), thereby obtaining 2-pentylheptyl 5-hydroxyundecanoate (48.9 g) as a colorless oily substance.

¹H-NMR(CDCl₃) δ: 3.98 (2H, d, J = 6.0 Hz), 3.63-3.57 (1H, m), 2.41-2.28 (2H, m), 1.84-1.22 (32H, m), 0.88 (9H, t, J = 7.2 Hz).

(3)

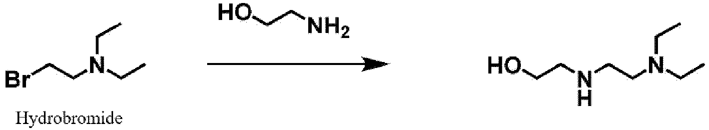

2-Bromo-N,N-diethylethan-1-amine hydrobromide (500.1 g) was added to a 1,2-dimethoxyethane (1.8 L) solution of 2-aminoethanol (351.5 g), and the mixture was stirred at 80°C for 2 hours. The reaction mixture was cooled to room temperature, and the solvent was distilled away under reduced pressure. Ethyl acetate (1 L) was added to the obtained residue, then a water (600 g) solution of potassium carbonate (540 g) was added thereto, and the mixture was stirred at room temperature for 30 minutes. After filtering off the insoluble matter, ethyl acetate (500 mL) was added thereto, the organic layer was separated, and the aqueous layer was extracted with ethyl acetate (1 L). The organic layer and the extraction solution were combined, and dried over anhydrous sodium sulfate (200 g), and then the solvent was distilled off under reduced pressure. The obtained residue was purified by distillation under reduced pressure to obtain 2-((2-(diethylamino)ethyl)amino)ethan-1-ol (109.2 g) as a colorless oily substance.

¹H-NMR(CDCl₃) δ: 3.65-3.61 (2H, m), 2.80-2.76 (2H, m), 2.69 (2H, t, J = 4.0 Hz), 2.56-2.50 (6H, m), 1.02 (6H, t, J = 8.0 Hz).

MS m/z (M + H): 161.

(4)

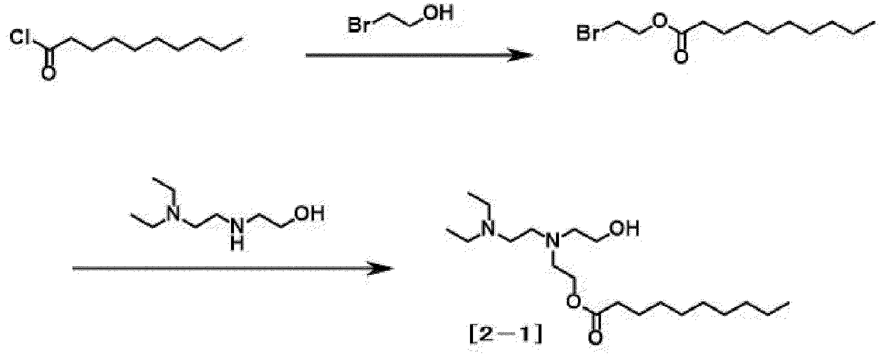

A mixture of triethylamine (28.1 g), 4-dimethylaminopyridine (0.16 g), and tetrahydrofuran (28 mL) was added dropwise to a mixture of decanoyl chloride (50.0 g), tetrahydrofuran (200 mL), and 2-bromoethan-1-ol (33.1 g) under ice cooling, and the mixture was stirred at room temperature for 3 hours. Water (250 mL) and toluene (750 mL) were added to the reaction mixture, and the organic layer was separated. The organic layer was washed with 2 mol/L hydrochloric acid (250 mL), water (250 mL), and a saturated aqueous sodium chloride solution (125 mL), dried over anhydrous sodium sulfate (150 g), and then the solvent was distilled off under reduced pressure. NH silica gel (50 g) and toluene (250 mL) were added to the residue, and the mixture was stirred at room temperature for 10 minutes. After filtering off the insoluble matter, the solvent was distilled off under reduced pressure to obtain 2-bromoethyl decanoate (69.0 g) as a light yellow oily substance.

¹H-NMR(CDCl₃) δ: 4.39 (2H, t, J = 6.2 Hz), 3.51 (2H, t, J = 6.2 Hz), 2.35 (2H, t, J = 7.6 Hz), 1.67-1.60 (2H, m), 1.33-1.26 (12H, m), 0.88 (3H, t, J = 6.8 Hz).

N,N-Dimethylformamide (50 mL) was added to the obtained 2-bromoethyl decanoate (40.0 g), and then 2-((2-(diethylamino)ethyl)amino)ethan-1-ol (27.8 g), powdered potassium carbonate (23.9 g), and potassium iodide (7.2 g), which pulverized using a mortar, were added thereto and stirred at 60°C for 4 hours. The reaction mixture was cooled to room temperature, then ethyl acetate (400 mL) and water (400 mL) were added thereto, and the organic layer was separated. The organic layer was washed with a 6% aqueous sodium hydrogen carbonate solution (200 mL) and a saturated aqueous sodium chloride solution (100 mL) and dried over anhydrous sodium sulfate (30.8 g), and then the solvent was distilled off under reduced pressure. The obtained residue was purified by silica gel column chromatography (NH silica gel) to obtain a compound [2-1] (40.1 g) as a colorless oily substance.

¹H-NMR(CDCl₃) δ: 4.15 (2H, t, J = 6.0 Hz), 3.54 (2H, t, J = 5.0 Hz), 2.84 (2H, t, J = 6.0 Hz), 2.70-2.65 (4H, m), 2.54 (4H, q, J = 7.2 Hz), 2.48 (2H, t, J = 6.0 Hz), 2.30 (2H, t, J = 7.6 Hz), 1.65-1.58 (2H, m), 1.34-1.20 (12H, m), 1.03 (6H, t, J = 7.2 Hz), 0.88 (3H, t, J = 7.0 Hz).

MS m/z (M + H): 359.

(5)

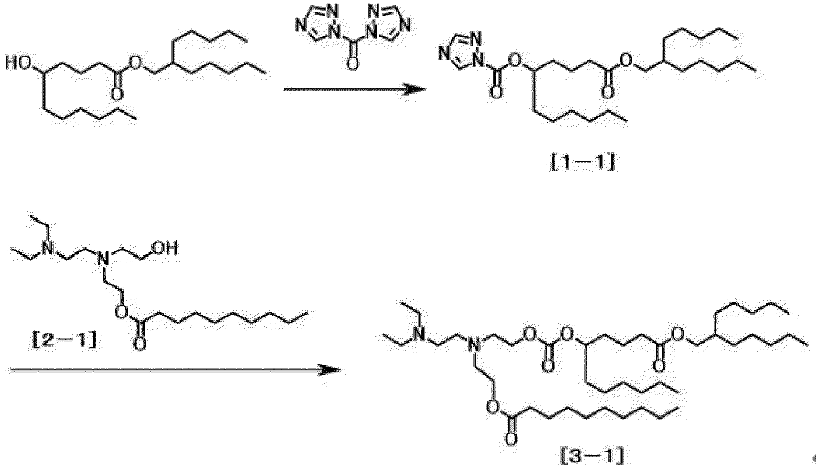

1,1'-Carbonyldi(1,2,4-triazole) (22.6 g) was added to a tetrahydrofuran (170 mL) solution of 2-pentylheptyl 5-hydroxyundecanoate (34.0 g), and the mixture was stirred at 30°C for 4 hours. Water (170 mL) was added dropwise to the reaction mixture, hexane (170 mL) and ethyl acetate (85 mL) were added thereto, and the organic layer was separated. The organic layer was washed with a 15% aqueous sodium chloride solution (200 mL), and dried over anhydrous sodium sulfate (34 g), and then the solvent was distilled off under reduced pressure to obtain a compound [1-1] (45.2 g).

¹H-NMR(CDCl₃) δ: 8.82 (1H, s), 8.07 (1H, s), 5.21-5.14 (1H, m), 3.97 (2H, d, J = 5.6 Hz), 2.36 (2H, t, J = 7.2 Hz), 1.88-1.55 (7H, m), 1.40-1.22 (24H, m), 0.91-0.85 (9H, m).

Tetrahydrofuran (83 mL), a tetrahydrofuran (68 mL) solution of the compound [2-1] (37.8 g), and triethylamine (18.6 g) were added to the obtained compound [1-1] (44.8 g), and the mixture was stirred at 50°C for 24 hours. The reaction mixture was cooled to 30°C, ethyl acetate (170 mL), hexane (170 mL), and water (170 mL) were added thereto, and the organic layer was separated. The organic layer was washed with water (170 mL) and a 20% aqueous sodium chloride solution (200 mL), and dried over anhydrous sodium sulfate (80.1 g), and then the solvent was distilled off under reduced pressure. The obtained residue was purified by silica gel column chromatography (ethyl acetate-hexane) to obtain a compound [3-1] (57.5 g, yield of 83% (two steps)) as a light yellow oily substance.

¹H-NMR(CDCl₃) δ: 4.72-4.65 (1H, m), 4.22-4.09 (4H, m), 3.97 (2H, d, J = 5.6 Hz), 2.86-2.77 (4H, m), 2.70-2.64 (2H, m), 2.55-2.48 (6H, m), 2.35-2.27 (4H, m), 1.71-1.55 (9H, m), 1.36-1.20 (36H, m), 1.02 (6H, t, J = 7.2 Hz), 0.94-0.82 (12H, m).

MS m/z (M + H): 755.

### [Example 2]

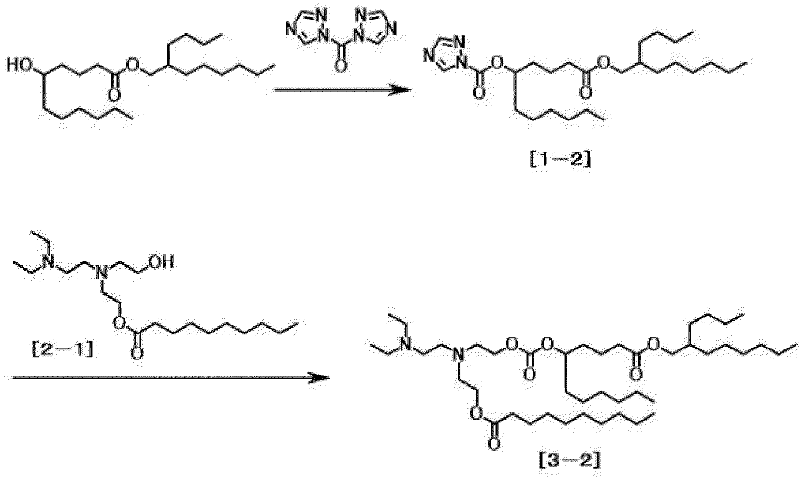

1,1'-Carbonyldi(1,2,4-triazole) (1.7 g) was added to a tetrahydrofuran (13 mL) solution of 2-butyloctyl 5-hydroxyundecanoate (2.5 g), and the mixture was stirred at 30°C for 3 hours. Water (52 mL) and hexane (52 mL) were added to the reaction mixture, and the organic layer was separated. The organic layer was washed with water (52 mL) and a saturated aqueous sodium chloride solution (52 mL), and dried over anhydrous sodium sulfate, and then the solvent was distilled off under reduced pressure to obtain a compound [1-2] (3.2 g).

¹H-NMR(CDCl₃) δ: 8.82 (1H, s), 8.07 (1H, s), 5.21-5.14 (1H, m), 3.97 (2H, d, J = 5.6 Hz), 2.36 (2H, t, J = 7.2 Hz), 1.88-1.55 (7H, m), 1.40-1.22 (24H, m), 0.91-0.85 (9H, m).

Tetrahydrofuran (15 mL), the compound [2-1] (2.9 g), and triethylamine (1.3 g) were added to the obtained compound [1-2] (3.0 g), and the mixture was stirred at 50°C for 24 hours. The reaction mixture was cooled to 30°C, ethyl acetate (30 mL) and water (30 mL) were added thereto, and the organic layer was separated. The organic layer was washed with water (30 mL) and a saturated aqueous sodium chloride solution (30 mL), and dried over anhydrous sodium sulfate, and then the solvent was distilled off under reduced pressure. The obtained residue was purified by silica gel column chromatography (ethyl acetate-hexane) to obtain a compound [3-2] (4.1 g, yield of 85% (two steps)) as a light yellow oily substance.

¹H-NMR(CDCl₃) δ: 4.72-4.65 (1H, m), 4.22-4.09 (4H, m), 3.97 (2H, d, J = 5.6 Hz), 2.86-2.77 (4H, m), 2.70-2.64 (2H, m), 2.55-2.48 (6H, m), 2.35-2.27 (4H, m), 1.71-1.55 (9H, m), 1.36-1.20 (36H, m), 1.02 (6H, t, J = 7.2 Hz), 0.94-0.82 (12H, m).

MS m/z (M + H): 755.

### [Example 3]

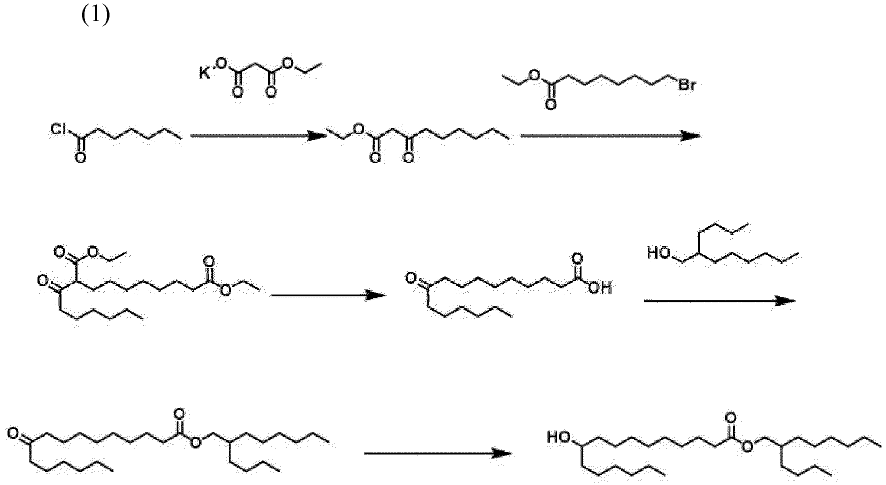

Triethylamine (102.1 g) and anhydrous magnesium chloride (80.1 g) were added to a mixture of potassium monoethyl malonate (114.5 g) and tetrahydrofuran (900 mL) under ice cooling, and the mixture was stirred at room temperature for 1 hour. Heptanoyl chloride (50.0 g) was added dropwise to the reaction mixture under ice cooling, and the mixture was stirred at room temperature for 3 hours. A water (1 L) solution of citric acid (193 g) was added dropwise to the reaction mixture under ice cooling, then toluene (500 mL) was added thereto, and the organic layer was separated. The organic layer was washed once with water (500 mL), twice with a 5% aqueous sodium hydrogen carbonate solution (500 mL), and once with a saturated aqueous sodium chloride solution (150 mL), and dried over anhydrous sodium sulfate (200 g), and then the solvent was distilled off under reduced pressure to obtain ethyl 3-oxononanoate (67.8 g) as a light yellow oily substance.

¹H-NMR(CDCl₃) δ: 4.20 (2H, q, J = 8.0 Hz), 3.43 (2H, s), 2.53 (2H, t, J = 8.0 Hz), 1.65-1.52 (2H, m), 1.26-1.21 (9H, m), 0.88 (3H, t, 8.0 Hz).

Ethanol (40 mL) was added to the obtained ethyl 3-oxononanoate (67.4 g), then a 20% sodium ethoxide-ethanol solution (117.8 g) was added thereto, ethyl 8-bromooctanoate (83.7 g) was added dropwise thereto, and the mixture was stirred at 90°C for 6 hours. The reaction mixture was cooled to room temperature, then a water (135 g) solution of sodium hydroxide (33.0 g) was added thereto, and the mixture was stirred at room temperature for 2 hours. A mixture of toluene (740 mL) and 30% hydrochloric acid (155 g) with water (135 g) was added to the reaction mixture, and the mixture was stirred at 60°C for 30 minutes. The reaction mixture was cooled to room temperature, and then the organic layer was separated. The organic layer was washed with water (335 mL) and a saturated aqueous sodium chloride solution (335 mL), and dried over anhydrous sodium sulfate (135 g), and then the solvent was distilled off under reduced pressure. Hexane (630 mL) was added to the obtained residue, and the mixture was stirred at 50°C for 1 hour and then stirred at 0°C for 1 hour. The solid matter was collected by filtration and then washed with hexane (250 mL) to obtain 10-oxohexadecanoic acid (36.0 g) as a light yellow solid.

¹H-NMR(CDCl₃) δ: 2.45-2.28 (6H, m), 1.71-1.47 (6H, m), 1.40-1.20 (14H, m), 0.88 (3H, t, J = 8.0 Hz).

p-Toluenesulfonic acid monohydrate (1.5 g) was added to a mixture of 10-oxohexadecanoic acid (25.0 g), 2-pentylheptane-1-ol (15.0 g), and toluene (105 mL), and the mixture was stirred under heating and reflux for 6 hours. After cooling the reaction mixture to room temperature, a saturated aqueous sodium hydrogen carbonate solution (105 mL) was added thereto, and the mixture was stirred for 5 minutes. The insoluble matter was removed by filtration through Celite, hexane (105 mL) and water (50 mL) were added thereto, and the organic layer was separated. The organic layer was washed with a saturated aqueous sodium hydrogen carbonate solution (105 mL) and a saturated aqueous sodium chloride solution (30 mL), and dried over anhydrous sodium sulfate (20 g), and the solvent was distilled off under reduced pressure to obtain 2-butyloctyl 10-oxohexadecanoate (34.1 g) as a light yellow oily substance.

¹H-NMR(CDCl₃) δ: 3.97 (2H, d, J = 5.8 Hz), 2.37 (4H, t, J = 7.4 Hz), 2.29 (2H, t, J = 7.5 Hz), 1.63-1.52 (7H, m), 1.40-1.19 (30H, m), 0.96-0.81 (9H, m).

Toluene (170 mL) was added to the obtained 2-butyloctyl 10-oxohexadecanoate (34.0 g), then sodium borohydride (2.2 g) was added thereto under ice cooling, methanol (22 mL) was added dropwise thereto at the same temperature, and the mixture was stirred at the same temperature for 3 hours. A mixture of hexane (170 mL) and 30% hydrochloric acid (6.8 mL) with water (160 mL) was added to the reaction mixture, and the organic layer was separated. The organic layer was washed twice with water (170 mL) and once with a saturated aqueous sodium chloride solution (64 mL), and dried over anhydrous sodium sulfate (34 g), and then the solvent was distilled off under reduced pressure. The obtained residue was purified by silica gel column chromatography (ethyl acetate-hexane), thereby obtaining 2-butyloctyl 10-hydroxyhexadecanoate (24.9 g) as a light yellow oily substance.

¹H-NMR(CDCl₃) δ: 3.97 (2H, d, J = 5.8 Hz), 3.63-3.52 (1H, m), 2.30 (2H, t, J = 7.5 Hz), 1.68-1.21 (42H, m), 0.96-0.81 (9H, m).

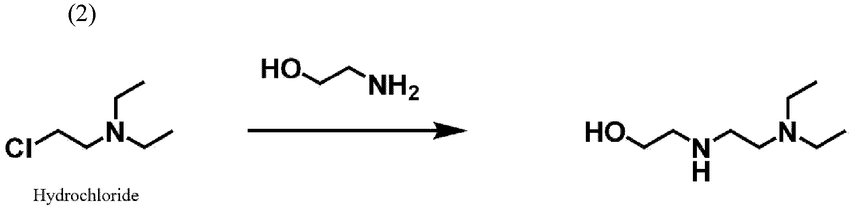

Ethanolamine (500 mL) was added to a mixture of 2-chloro-N,N-diethylethan-1-amine hydrochloride (520 g) and 1,4-dioxane (2 L), and the mixture was stirred under heating and reflux for 4 hours. The solvent of the reaction mixture was distilled off under reduced pressure, ethyl acetate (2 L) was added to the residue, then a 45% aqueous potassium carbonate solution (680 mL) was added thereto, and the mixture was stirred for 30 minutes. The insoluble matter was filtered off, and the solvent was distilled off under reduced pressure. The obtained residue was purified by distillation under reduced pressure to obtain 2-((2-(diethylamino)ethyl)amino)ethan-1-ol (300 g) as a colorless oily substance.

¹H-NMR(CDCl₃) δ: 3.65-3.61 (2H, m), 2.80-2.76 (2H, m), 2.69 (2H, t, J = 4.0 Hz), 2.56-2.50 (6H, m), 1.02 (6H, t, J = 8.0 Hz).

MS m/z(M + H): 161.

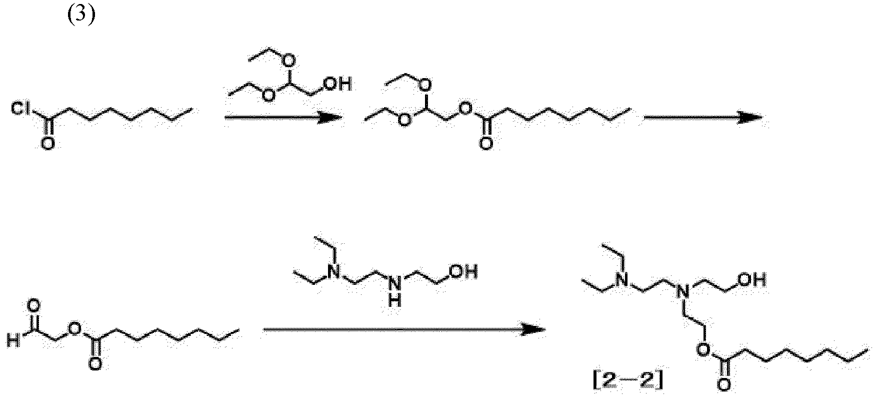

Octanoyl chloride (72.7 g) was added dropwise to a mixture of 2,2-diethoxyethan-1-ol (50.0 g), triethylamine (49.0 g), and toluene (500 mL) under ice cooling, and the mixture was stirred at room temperature for 2 and a half hours. Water (500 mL) was added dropwise to the reaction mixture under ice cooling, and then the organic layer was separated. After washing with a 10% aqueous potassium carbonate solution (500 mL), water (200 mL), and a 20% aqueous sodium chloride solution (250 mL), NH silica gel (97.0 g) and anhydrous sodium sulfate (200 g) were added thereto, and the mixture was stirred for 1 hour. After filtering off the insoluble matter, the solvent was distilled off under reduced pressure to obtain 2,2-diethoxyethyl octanoate (98.6 g) as a yellow oily substance.

¹H-NMR(CDCl₃) δ: 4.68 (1H, t, J = 4.0 Hz), 4.10 (2H, d, J = 4.0 Hz), 3.72 (1H, q, J = 6.8 Hz), 3.69 (1H, q, J = 6.8 Hz, 3.58 (1H, q, J = 6.8 Hz), 3.56 (1H, q, J = 6.8 Hz), 2.34 (2H, t, J = 7.6 Hz), 1.67-1.57 (2H, m), 1.37-1.21 (8H, m), 1.22 (6H, t, J = 6.8 Hz), 0.88 (3H, t, J = 7.2 Hz).

Formic acid (80 mL) was added to a mixture of 2,2-diethoxyethyl octanoate (40.0 g) and water (20 mL), and the mixture was stirred at 30°C for 3 hours. A water (400 mL) solution of ethyl acetate (600 mL) and sodium chloride (100 g) was added to the reaction mixture, and the organic layer was separated. The organic layer was washed twice with a mixture of potassium carbonate (80 g), sodium chloride (20 g), and water (400 g) and once with a water (400 mL) solution of sodium chloride (100 g), then anhydrous sodium sulfate (200 g) was added thereto, and the mixture was stirred at room temperature for 2 hours. The insoluble matter was filtered off to obtain an ethyl acetate solution of 2-oxoethyl octanoate.

¹H-NMR(CDCl₃) δ: 9.61 (1H, s), 4.67 (2H, s), 2.45 (2H, t, J = 7.6 Hz), 1.76-1.57 (2H, m), 1.39-1.19 (8H, m), 0.88 (3H, t, J = 7.2 Hz).

An ethyl acetate (100 mL) solution of 2-((2-(diethylamino)ethyl)amino)ethan-1-ol (29.1 g) was added dropwise to the obtained ethyl acetate solution of 2-oxoethyl octanoate under ice cooling, then sodium triacetoxyborohydride (48.1 g) was added thereto, and the mixture was stirred at room temperature for 2 hours. A water (800 g) solution of potassium carbonate (155.3 g) was added dropwise to the reaction mixture under ice cooling, and then the organic layer was separated. The organic layer was washed with a water (800 g) solution of potassium carbonate (80 g) and a water (200 g) solution of sodium chloride (60 g), then 1% hydrochloric acid (800 g) was added thereto, the aqueous layer was separated, and the obtained aqueous layer was washed with ethyl acetate (200 mL). The obtained hydrochloric acid mixture was added dropwise to a mixture of ethyl acetate (500 mL), potassium carbonate (90.3 g), and water (600 g) under ice cooling, then the organic layer was separated and washed with a saturated aqueous sodium chloride solution (150 mL), anhydrous sodium sulfate (200 g) was added thereto, and the mixture was stirred at room temperature for 1 hour. After filtering off the insoluble matter, the solvent was distilled off under reduced pressure, and the obtained residue was purified by silica gel column chromatography (NH silica gel, ethyl acetate-hexane) to obtain a compound [2-2] (30.2 g) as a colorless oily substance.

¹H-NMR(CDCl₃) δ: 5.20 (1H, brs), 4.15 (2H, t, J = 6.0 Hz), 3.54 (2H, t, J = 4.8 Hz), 2.84 (2H, t, J = 6.0 Hz), 2.70-2.64 (4H, m), 2.54 (4H, q, J = 7.2 Hz), 2.47 (2H, t, J = 6.0 Hz), 2.30 (2H, t, J = 7.6 Hz), 1.66-1.55 (2H, m), 1.34-1.22 (8H, m), 1.03 (6H, t, J = 7.2 Hz), 0.88 (3H, t, J = 7.0 Hz).

MS m/z (M + H): 331.

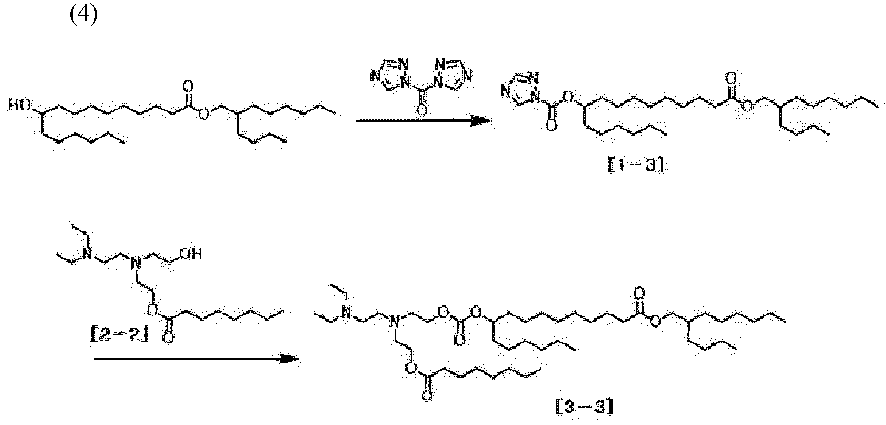

1,1'-Carbonyldi(1,2,4-triazole) (5.9 g) was added to a tetrahydrofuran (53 mL) solution of 2-butyloctyl 10-hydroxyhexadecanoate (10.6 g), and the mixture was stirred at 30°C for 3 hours. Water (50 mL), ethyl acetate (50 mL), and hexane (50 mL) were added to the reaction mixture, and the organic layer was separated. The organic layer was washed with water (50 mL) and a saturated aqueous sodium chloride solution (25 mL), and dried over anhydrous sodium sulfate (40 g), and then the solvent was distilled off under reduced pressure to obtain a compound [1-3] (13.5 g).

¹H-NMR(CDCl₃) δ: 8.82 (1H, s), 8.07 (1H, s), 5.21-5.14 (1H, m), 3.97 (2H, d, J = 5.6 Hz), 2.36 (2H, t, J = 7.2 Hz), 1.88-1.55 (7H, m), 1.40-1.22 (34H, m), 0.91-0.85 (9H, m).

Tetrahydrofuran (52 mL), the compound [2-2] (8.8 g), and triethylamine (4.9 g) were added to the obtained compound [1-3] (13.0 g), and the mixture was stirred at 50°C for 25 hours. The reaction mixture was cooled to 30°C, ethyl acetate (52 mL), hexane (52 mL), and water (52 mL) were added thereto, and the organic layer was separated. The organic layer was washed with water (52 mL) and a saturated aqueous sodium chloride solution (52 mL), and dried over anhydrous sodium sulfate, and then the solvent was distilled off under reduced pressure. The obtained residue was purified by silica gel column chromatography (ethyl acetate-hexane) to obtain a compound [3-3] (14.9 g, yield of 77% (two steps)) as a light yellow oily substance.

¹H-NMR(CDCl₃) δ: 4.71-4.62 (1H, m), 4.20-4.08 (4H, m), 3.97 (2H, d, J = 5.6 Hz), 2.89-2.77 (4H, m), 2.73-2.42 (8H, m), 2.29 (4H, t, J = 7.6 Hz), 1.68-1.48 (9H, m), 1.39-1.18 (42H, m), 1.10-0.98 (6H, m), 0.94-0.81 (12H, m).

MS m/z (M + H): 798.

### [Example 4]

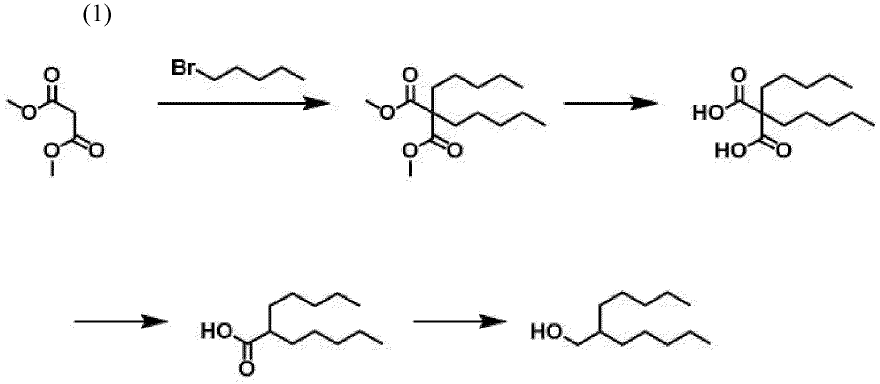

Dimethyl malonate (100.0 g) was added to a mixture of dimethyl sulfoxide (750 mL), toluene (750 mL), and powdered potassium hydroxide (109.9 g) under ice cooling, the mixture was stirred at room temperature for 10 minutes, then 1-bromopentane (274.4 g) was added thereto, and the mixture was stirred at room temperature for 5 hours. 7% hydrochloric acid (900 mL) was added dropwise to the reaction mixture under ice cooling, and then the organic layer was separated and washed with water (200 mL) and a 10% aqueous sodium chloride solution. After drying over anhydrous sodium sulfate, the solvent was distilled off under reduced pressure to obtain dimethyl 2,2-dipentylmalonate (201.6 g) as a light yellow oily substance.

¹H-NMR(CDCl₃) δ: 3.70 (6H, s), 1.89-1.83 (4H, m), 1.35-1.22 (8H, m), 1.17-1.08 (4H, m), 0.87 (6H, t, J = 7.0 Hz).

A water (430 mL) solution of tetrahydrofuran (430 mL), methanol (430 mL), and potassium hydroxide (260 g) was added to the obtained dimethyl 2,2-dipentylmalonate (179.0 g), and the mixture was stirred at 70°C for 34 hours. The reaction mixture was cooled to room temperature, 15% hydrochloric acid (880 mL) was added dropwise thereto under ice cooling, then ethyl acetate (420 mL) was added thereto, and the organic layer was separated. The organic layer was washed with a 5% aqueous sodium hydrogen carbonate solution (420 mL), and dried over anhydrous sodium sulfate (100 g), and then the solvent was distilled off under reduced pressure. Hexane (480 mL) and ethyl acetate (48 mL) were added to the obtained residue, and the mixture was stirred under ice cooling for 1 hour. The solid matter was collected by filtration and then washed with hexane (200 mL) to obtain 2,2-dipentylmalonic acid (105.1 g) as a white solid.

¹H-NMR(CDCl₃) δ: 1.98-1.91 (4H, m), 1.35-1.22 (12H, m), 0.87 (6H, t, J = 7.0 Hz).

A mixture of 2,2-dipentylmalonic acid (100.0 g) and pyridine (500 mL) was stirred under heating and reflux for 8 hours. The solvent of the reaction mixture was distilled off under reduced pressure, ethyl acetate (200 mL), hexane (200 mL), and 10% hydrochloric acid (200 mL) were added to the obtained residue, and the organic layer was separated. The organic layer was washed with water (200 mL) and dried over anhydrous sodium sulfate (20 g), and the solvent was distilled off under reduced pressure to obtain 2-pentylheptanoic acid (81.6 g) as a light yellow oily substance.

¹H-NMR(CDCl₃) δ: 2.39-2.31 (1H, m), 1.67-1.56 (2H, m), 1.52-1.42 (2H, m), 1.36-1.22 (12H, m), 0.90-0.85 (6H, m).

Isobutyl chloroformic acid (6.5 g) was added dropwise to a mixture of 2-pentylheptanoic acid (8.5 g), 4-methylmorpholine (5.2 g), and tetrahydrofuran (42.5 mL) under ice cooling, and the mixture was stirred at the same temperature for 30 minutes. Water (42.5 mL) was added to the reaction mixture, the organic layer was separated and then washed with water (42.5 mL), and a tetrahydrofuran mixture was obtained. Tetrahydrofuran (21.3 mL) was added to the obtained tetrahydrofuran mixture, then sodium borohydride (3.53 g) was added thereto under ice cooling, the mixture was stirred at the same temperature for 10 minutes, then methanol (12.8 mL) was added thereto, and the mixture was stirred at room temperature for 1 hour. A 10% aqueous ammonium chloride solution (80 mL) was added to the reaction mixture under ice cooling, the mixture was stirred at room temperature for 5 minutes, then hexane (42.5 mL) was added thereto, and the organic layer was separated. The organic layer was washed with water and dried over anhydrous sodium sulfate, and then the solvent was distilled off under reduced pressure. The obtained residue was purified by distillation under reduced pressure to obtain 2-pentylheptan-1-ol (3.7 g) as a light yellow oily substance.

¹H-NMR(CDCl₃) δ: 3.57-3.51 (2H, m), 1.50-1.20 (17H, m), 0.88 (6H, t, J = 6.6 Hz).

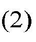

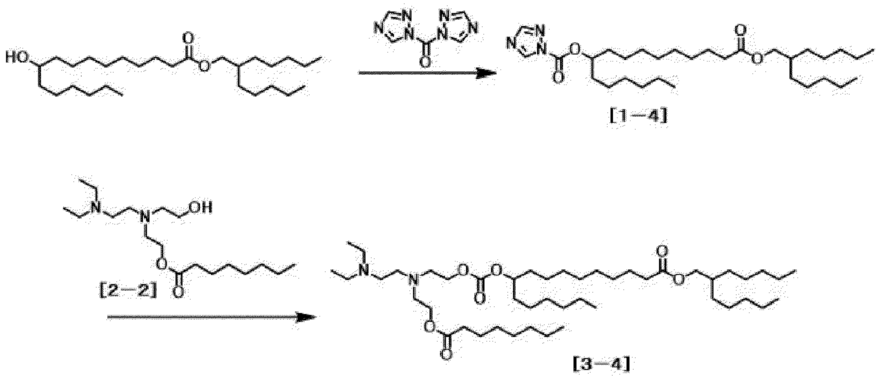

1,1'-Carbonyldi(1,2,4-triazole) (3.8 g) was added to a tetrahydrofuran (34 mL) solution of 2-pentylheptyl 10-hydroxyhexadecanoate (6.9 g), and the mixture was stirred at 30°C for 5 hours. Water (35 mL), ethyl acetate (20 mL), and hexane (35 mL) were added to the reaction mixture, and the organic layer was separated. The organic layer was washed twice with water (35 mL) and once with a saturated aqueous sodium chloride solution (35 mL), and dried over anhydrous sodium sulfate, and then the solvent was distilled off under reduced pressure to obtain a compound [1-4] (8.3 g).

¹H-NMR(CDCl₃) δ: 8.82 (1H, s), 8.07 (1H, s), 5.21-5.14 (1H, m), 3.97 (2H, d, J = 5.6 Hz), 2.36 (2H, t, J = 7.2 Hz), 1.88-1.55 (7H, m), 1.40-1.22 (34H, m), 0.91-0.85 (9H, m).

Tetrahydrofuran (41 mL), the compound [2-2] (5.6 g), and triethylamine (3.1 g) were added to the obtained compound [1-4] (8.3 g), and the mixture was stirred at 55°C for 27 hours. The reaction mixture was cooled to 30°C, ethyl acetate (40 mL), hexane (40 mL), and water (40 mL) were added thereto, and the organic layer was separated. The organic layer was washed with water (40 mL) and a saturated aqueous sodium chloride solution (40 mL), and dried over anhydrous sodium sulfate, and then the solvent was distilled off under reduced pressure. The obtained residue was purified by silica gel column chromatography (ethyl acetate-hexane) to obtain a compound [3-4] (9.8 g, yield of 79% (two steps)) as a light yellow oily substance.

¹H-NMR(CDCl₃) δ: 4.73-4.61 (1H, m), 4.21-4.07 (4H, m), 3.97 (2H, d, J = 5.4 Hz), 2.88-2.77 (4H, m), 2.72-2.62 (2H, m), 2.58-2.45 (6H, m), 2.29 (4H, t, J = 7.2 Hz), 1.69-1.48 (9H, m), 1.41-1.18 (42H, m), 1.02 (6H, t, J = 7.2 Hz), 0.94-0.82 (12H, m).

MS m/z (M + H): 798.

### [Example 5]

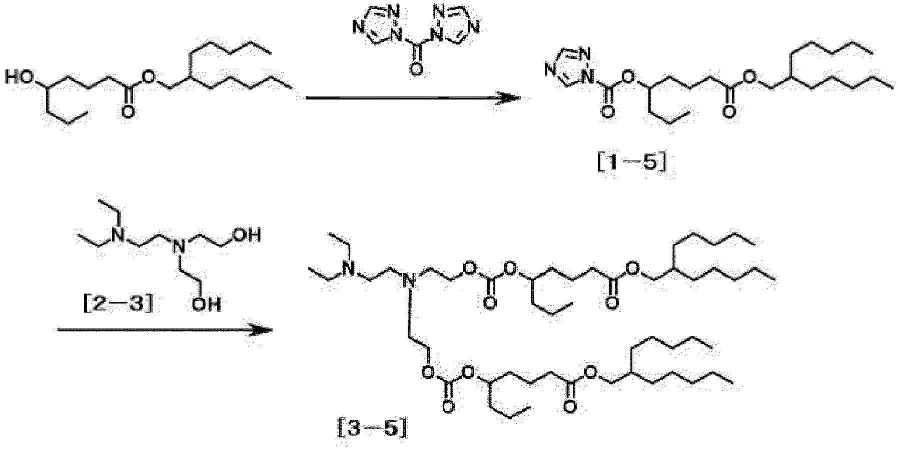

1,1'-Carbonyldi(1,2,4-triazole) (1.4 g) was added to a tetrahydrofuran (9 mL) solution of 2-pentylheptyl 5-hydroxyoctanoate (1.8 g), and the mixture was stirred at 30°C for 2 hours. Water (15 mL), ethyl acetate (3 mL), and hexane (15 mL) were added to the reaction mixture, and the organic layer was separated. The organic layer was washed with water (15 mL) and a saturated aqueous sodium chloride solution (15 mL), and dried over anhydrous sodium sulfate, and then the solvent was distilled off under reduced pressure to obtain a compound [1-5] (2.3 g).

¹H-NMR(CDCl₃) δ: 8.82 (1H, s), 8.07 (1H, s), 5.21-5.14 (1H, m), 3.97 (2H, d, J = 5.6 Hz), 2.36 (2H, t, J = 7.2 Hz), 1.88-1.55 (7H, m), 1.40-1.22 (18H, m), 0.91-0.85 (9H, m).

Acetonitrile (7 mL), the compound [2-3] (0.50 g), and 1,8-diazabicyclo[5.4.0]-7-undecene (1.3 g) were added to the obtained compound [1-5] (2.3 g), and the mixture was stirred at 50°C for 2 hours. The reaction mixture was cooled to 30°C, ethyl acetate (70 mL) and water (70 mL) were added thereto, and the organic layer was separated. The organic layer was washed with water (35 mL) and a saturated aqueous sodium chloride solution (35 mL), and dried over anhydrous sodium sulfate, and then the solvent was distilled off under reduced pressure. The obtained residue was purified by silica gel column chromatography (ethyl acetate-hexane) to obtain a compound [3-5] (1.6 g, yield of 73%) as a light yellow oily substance.

¹H-NMR(CDCl₃) δ: 4.74-4.67 (2H, m), 4.22-4.10 (4H, m), 3.97 (4H, d, J = 5.6 Hz), 2.83 (4H, t, J = 6.4 Hz), 2.69-2.65 (2H, m), 2.55-2.49 (6H, m), 2.34-2.30 (4H, m), 1.74-1.48 (14H, m), 1.43-1.20 (36H, m), 1.01 (6H, t, J = 7.2 Hz), 0.93-0.87 (18H, m).

MS m/z (M + H): 914.

### [Example 6]

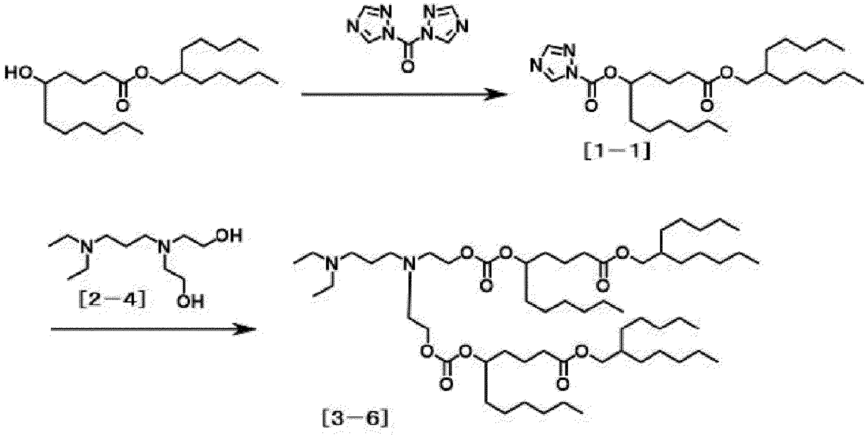

1,1'-Carbonyldi(1,2,4-triazole) (0.66 g) was added to a tetrahydrofuran (5 mL) solution of 2-pentylheptyl 5-hydroxyundecanoate (1.0 g), and the mixture was stirred at 30°C for 2 hours. Water (10 mL), ethyl acetate (2 mL), and hexane (10 mL) were added to the reaction mixture, and the organic layer was separated. The organic layer was washed with water (10 mL) and a saturated aqueous sodium chloride solution (10 mL), and dried over anhydrous sodium sulfate, and then the solvent was distilled off under reduced pressure to obtain a compound [1-1] (1.2 g).

¹H-NMR(CDCl₃) δ: 8.82 (1H, s), 8.07 (1H, s), 5.21-5.14 (1H, m), 3.97 (2H, d, J = 5.6 Hz), 2.36 (2H, t, J = 7.2 Hz), 1.88-1.55 (7H, m), 1.40-1.22 (24H, m), 0.91-0.85 (9H, m).

Acetonitrile (4 mL), the compound [2-4] (0.26 g), and 1,8-diazabicyclo[5.4.0]-7-undecene (0.60 g) were added to the obtained compound [1-1] (1.2 g), and the mixture was stirred at 55°C for 2 hours. The reaction mixture was cooled to 30°C, ethyl acetate (40 mL) and water (40 mL) were added thereto, and the organic layer was separated. The organic layer was washed with water (20 mL) and a saturated aqueous sodium chloride solution (20 mL), and dried over anhydrous sodium sulfate, and then the solvent was distilled off under reduced pressure. The obtained residue was purified by silica gel column chromatography (ethyl acetate-hexane) to obtain a compound [3-6] (1.1 g, yield of 88%) as a light yellow oily substance.

¹H-NMR(CDCl₃) δ: 4.72-4.65 (2H, m), 4.22-4.09 (4H, m), 3.97 (4H, d, J = 6.0 Hz), 2.80 (4H, t, J = 6.4 Hz), 2.61-2.45 (6H, m), 2.44-2.37 (2H, m), 2.32 (4H, t, J = 7.2 Hz), 1.74-1.49 (16H, m), 1.35-1.21 (48H, m), 1.00 (6H, t, J = 7.2 Hz), 0.91-0.85 (18H, m).

MS m/z (M + H): 1012.

### [Example 7]

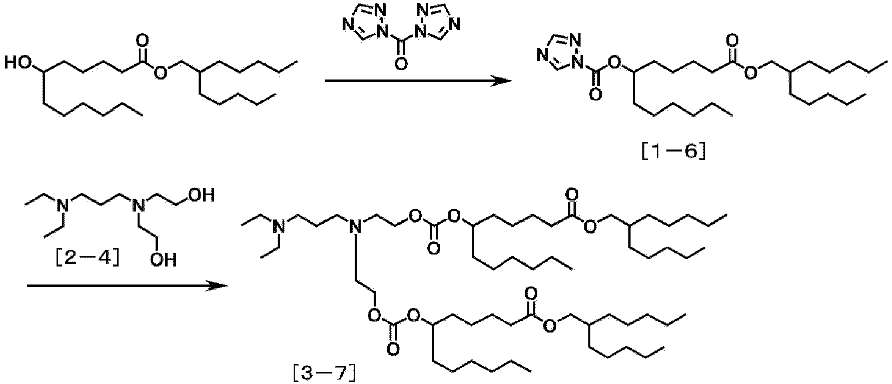

1-Oxo-1-((2-pentylheptyl)oxy)dodecan-6-yl 1H-1,2,4-triazole-1-carboxylate (compound [1-6]) was obtained by the same method as in [Example 6], using 2-pentylheptyl 6-hydroxydodecanoate as a raw material obtained by the same method as in [Example 1](2).

¹H-NMR(CDCl₃) δ: 8.81 (1H,s), 8.07 (1H, s), 5.20-5.12 (1H, m), 3.95 (2H, d, J = 5.8 Hz), 2.31 (2H, t, J = 7.42 Hz), 1.87-1.52 (7H, m), 1.48-1.19 (26H, m), 0.92-0.83 (9H, m).

Bis(2-pentylheptyl) 12-(3-(diethylamino)propyl)-6,18-dihexyl-8,16-dioxo-7,9,15,17-tetraoxa-12-azatricosanedioat e (compound [3-7] (0.34 g, yield of 89%)) was obtained by the same method as in [Example 6] using the obtained compound [1-6] (0.44 g) as a raw material.

¹H-NMR(CDCl₃) δ: 4.71-4.62 (2H, m), 4.22-4.08 (4H, m), 3.96 (4H, d, J = 5.8 Hz), 2.80 (4H, t, J = 6.4 Hz), 2.60-2.45 (6H, m), 2.44-2.37 (2H, m), 2.30 (4H, t, J = 7.5 Hz), 1.69-1.49 (16H, m), 1.44-1.18 (52H, m), 1.00 (6H, t, J = 7.1 Hz), 0.93-0.80 (18H, m).

MS m/z (M + H): 1040.

### [Example 8]

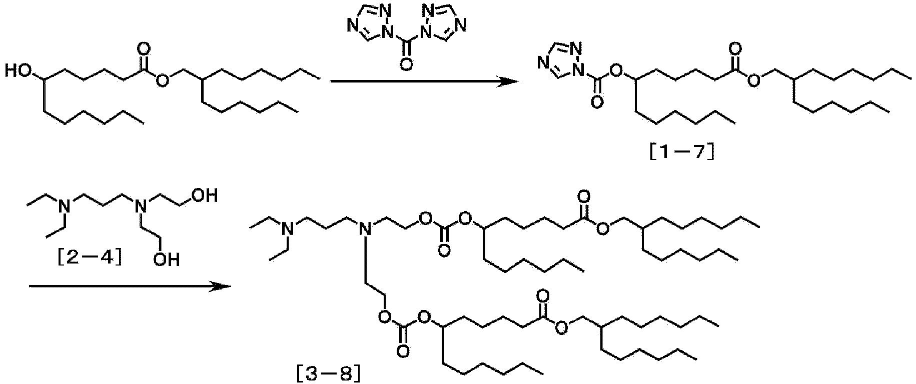

1-((2-Hexyloctyl)oxy)-1-oxododecan-6-yl 1H-1,2,4-triazole-1-carboxylate (compound [1-7]) was obtained by the same method as in [Example 6], using 2-hexyloctyl 6-hydroxydodecanoate as a raw material obtained by the same method as in [Example 1] (2).

¹H-NMR(CDCl₃) δ: 8.81 (1H, s), 8.07 (1H, s), 5.20-5.12 (1H, m), 3.95 (2H, d, J = 5.8 Hz), 2.31 (2H, t, J = 7.4 Hz), 1.88-1.53 (7H, m), 1.50-1.19 (30H, m), 0.92-0.82 (9H, m).

Bis(2-hexyloctyl) 12-(3-(diethylamino)propyl)-6,18-dihexyl-8,16-dioxo-7,9,15,17-tetraoxa-12-azatricosanedioat e (compound [3-8] (0.32 g, yield of 80%)) was obtained by the same method as in [Example 6] using the obtained compound [1-7] (0.46 g) as a raw material.

¹H-NMR(CDCl₃) δ: 4.70-4.62 (2H, m), 4.22-4.08 (4H, m), 3.96 (4H, d, J = 5.8 Hz), 2.80 (4H, t, J = 6.4 Hz), 2.60-2.46 (6H, m), 2.44-2.37 (2H, m), 2.30 (4H, t, J = 7.5 Hz), 1.69-1.49 (16H, m), 1.44-1.19 (60H, m), 1.00 (6H, t, J = 7.1 Hz), 0.93-0.81 (18H, m).

MS m/z (M + H): 1096.

### [Example 9]

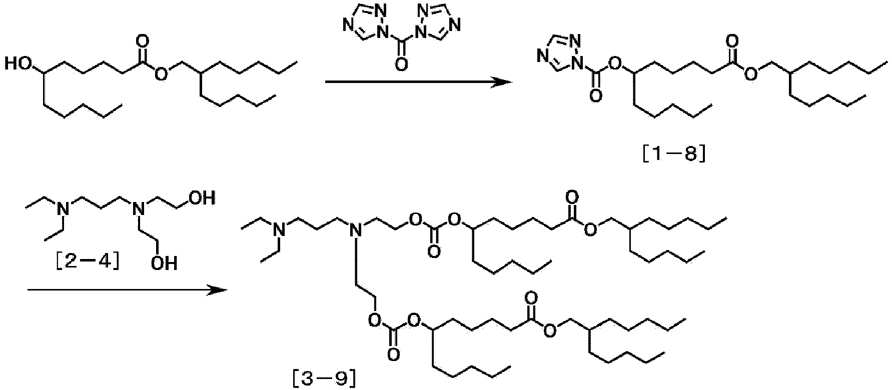

1-Oxo-1-((2-pentylheptyl)oxy)undecan-6-yl 1H-1,2,4-triazole-1-carboxylate (compound [1-8]) was obtained by the same method as in [Example 6], using 2-pentylheptyl 6-hydroxyundecanoate as a raw material obtained by the same method as in [Example 1](2).

¹H-NMR(CDCl₃) δ: 8.81 (1H, s), 8.07 (1H, s), 5.20-5.13 (1H, m), 3.95 (2H, d, J = 5.81 Hz), 2.31 (2H, t, J = 7.4 Hz), 1.90-1.51 (7H, m), 1.50-1.19 (24H, m), 0.95-0.82 (9H, m).

Bis(2-pentylheptyl) 12-(3-(diethylamino)propyl)-8,16-dioxo-6,18-dipentyl-7,9,15,17-tetraoxa-12-azatricosanedioat e (compound [3-9] (88 mg, yield of 63%)) was obtained by the same method as in [Example 6] using the obtained compound [1-8] (140 mg) as a raw material.

¹H-NMR(CDCl₃) δ: 4.72-4.61 (2H, m), 4.22-4.07 (4H, m), 3.96 (4H, d, J = 5.8 Hz), 2.80 (4H, t, J = 6.4 Hz), 2.59-2.45 (6H, m), 2.44-2.36 (2H, m), 2.30 (4H, t, J = 7.5 Hz), 1.70-1.48 (16H, m), 1.44-1.19 (48H, m), 1.00 (6H, t, J = 7.1 Hz), 0.92-0.81 (18H, m).

MS m/z (M + H): 1012.

### [Example 10]

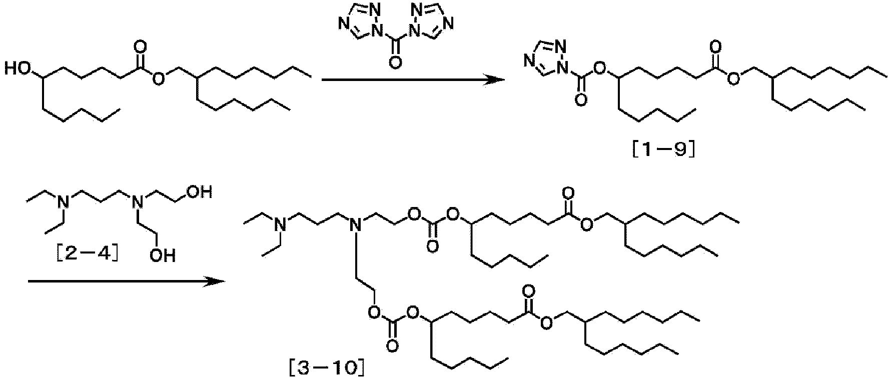

1-((2-Hexyloctyl)oxy)-1-oxaundecan-6-yl 1H-1,2,4-triazole-1-carboxylate (compound [1-9]) was obtained by the same method as in [Example 6], using 2-hexyloctyl 6-hydroxyundecanoate as a raw material obtained by the same method as in [Example 1](2).

¹H-NMR(CDCl₃) δ: 8.81 (1H s), 8.07 (1H, s), 5.22-5.11 (1H, m), 3.95 (2H, d, J = 5.8 Hz), 2.31 (2H, t, J = 7.4 Hz), 1.88-1.51 (7H, m), 1.50-1.19 (28H, m), 0.94-0.82 (9H, m).

Bis(2-hexyloctyl) 12-(3-(diethylamino)propyl)-8,16-dioxo-6,18-dipentyl-7,9,15,17-tetraoxa-12-azatricosanedioat e (compound [3-10] (0.31 g, yield of 90%)) was obtained by the same method as in [Example 6] using the obtained compound [1-9] (0.38 g) as a raw material.

¹H-NMR(CDCl₃) δ: 4.72-4.62 (2H, m), 4.22-4.07 (4H, m), 3.96 (4H, d, J = 5.8 Hz), 2.80 (4H, t, J = 6.4 Hz), 2.60-2.45 (6H, m), 2.44-2.36 (2H, m), 2.30 (4H, t, J = 7.5 Hz), 1.69-1.46 (16H, m), 1.44-1.18 (56H, m), 1.00 (6H, t, J = 7.1 Hz), 0.94-0.80 (18H, m).

MS m/z (M + H): 1068.

### [Comparative Example 1]

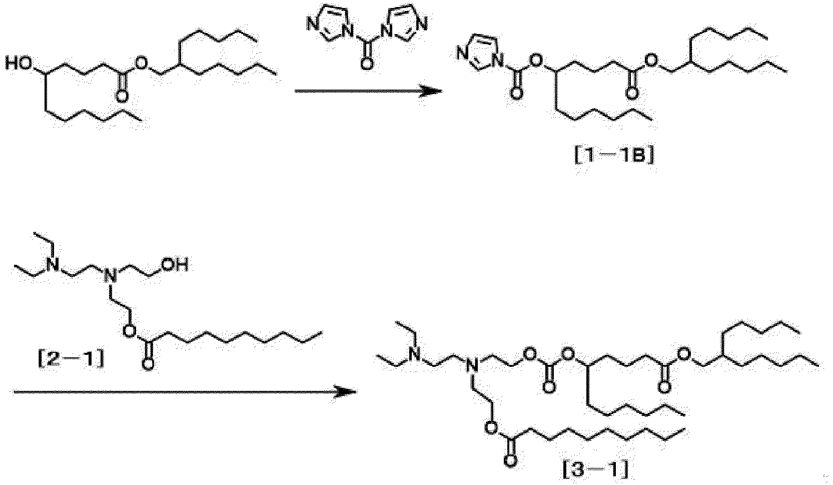

1,1'-Carbonyldiimidazole (328 mg) was added to a tetrahydrofuran (5.0 mL) solution of 2-pentylheptyl 5-hydroxyundecanoate (500 mg), and the mixture was stirred at room temperature for 30 minutes. Water (10 mL) and hexane (20 mL) were added to the reaction mixture, and the organic layer was separated, and then washed with water and a saturated aqueous sodium chloride solution. After drying over anhydrous sodium sulfate, the solvent was distilled off under reduced pressure to obtain a compound [1-1B] (636 mg) as a light yellow oily substance.

¹H-NMR(CDCl₃) δ: 8.14-8.12 (1H, m), 7.43-7.41 (1H, m), 7.08-7.06 (1H, m), 5.11-5.04 (1H, m), 3.97 (2H, d, J = 5.6 Hz), 2.38-2.32 (2H, m), 1.80-1.54 (7H, m), 1.40-1.22 (24H, m), 0.91-0.85 (9H, m).

Acetonitrile (2.5 mL), the compound [2-1] (564 mg), and potassium carbonate (208 mg) were added to the obtained compound [1-1B] (636 mg), and the mixture was stirred at 80°C for 2 hours. The reaction mixture was cooled to room temperature, then ethyl acetate (5 mL) was added thereto, the insoluble matters were filtered off, and the solvent was distilled away under reduced pressure. Ethyl acetate (20 mL) and water (20 mL) were added thereto, and the organic layer was separated. The organic layer was washed with water and a saturated aqueous sodium chloride solution, and dried over anhydrous sodium sulfate, and the solvent was distilled off under reduced pressure. The obtained residue was purified by silica gel column chromatography (methanol-ethyl acetate-hexane) to obtain a compound [3-1] (676 mg, yield of 65% (two steps)) as a colorless oily substance.

¹H-NMR(CDCl₃) δ: 4.72-4.65 (1H, m), 4.22-4.09 (4H, m), 3.97 (2H, d, J = 5.6 Hz), 2.86-2.77 (4H, m), 2.70-2.64 (2H, m), 2.55-2.48 (6H, m), 2.35-2.27 (4H, m), 1.71-1.55 (9H, m), 1.36-1.20 (36H, m), 1.02 (6H, t, J = 7.2 Hz), 0.94-0.82 (12H, m).

MS m/z (M + H): 755.

### [Comparative Example 2]

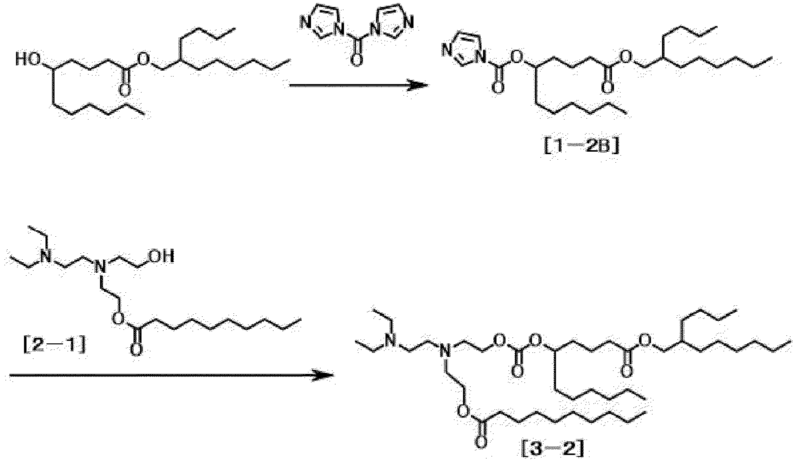

1,1'-Carbonyldiimidazole (1.3 g) was added to a tetrahydrofuran (10 mL) solution of 2-butyloctyl 5-hydroxyundecanoate (2.0 g), and the mixture was stirred at room temperature for 1 hour. Water (10 mL) and hexane (10 mL) were added to the reaction mixture, and the organic layer was separated, and then washed with water and a saturated aqueous sodium chloride solution. After drying over anhydrous sodium sulfate, the solvent was distilled off under reduced pressure to obtain a compound [1-2B] (3.0 g) as a light yellow oily substance.

¹H-NMR(CDCl₃) δ: 8.14-8.12 (1H, m), 7.43-7.41 (1H, m), 7.08-7.06 (1H, m), 5.11-5.04 (1H, m), 3.97 (2H, d, J = 5.6 Hz), 2.38-2.32 (2H, m), 1.80-1.54 (7H, m), 1.40-1.22 (24H, m), 0.91-0.85 (9H, m).

Acetonitrile (10 mL), the compound [2-1] (2.2 g), and potassium carbonate (1.6 g) were added to the obtained compound [1-2B] (3.0 g), and the mixture was stirred at 80°C for 2 hours. The reaction mixture was cooled to room temperature, then ethyl acetate (20 mL) and water (20 mL) were added thereto, the organic layer was separated, and the solvent was distilled off under reduced pressure. Ethyl acetate (10 mL), hexane (10 mL), water (10 mL), and methanol were added to the obtained residue, and the organic layer was separated. The organic layer was dried over anhydrous sodium sulfate, and then the solvent was distilled off under reduced pressure. The obtained residue was purified by silica gel column chromatography (methanol-ethyl acetate-hexane) to obtain a compound [3-2] (2.7 g, yield of 66% (two steps)) as a colorless oily substance.

¹H-NMR(CDCl₃) δ: 4.72-4.65 (1H, m), 4.22-4.09 (4H, m), 3.97 (2H, d, J = 5.6 Hz), 2.86-2.77 (4H, m), 2.70-2.64 (2H, m), 2.55-2.48 (6H, m), 2.35-2.27 (4H, m), 1.71-1.55 (9H, m), 1.36-1.20 (36H, m), 1.02 (6H, t, J = 7.2 Hz), 0.94-0.82 (12H, m).

MS m/z (M + H): 755.

### [Comparative Example 3]

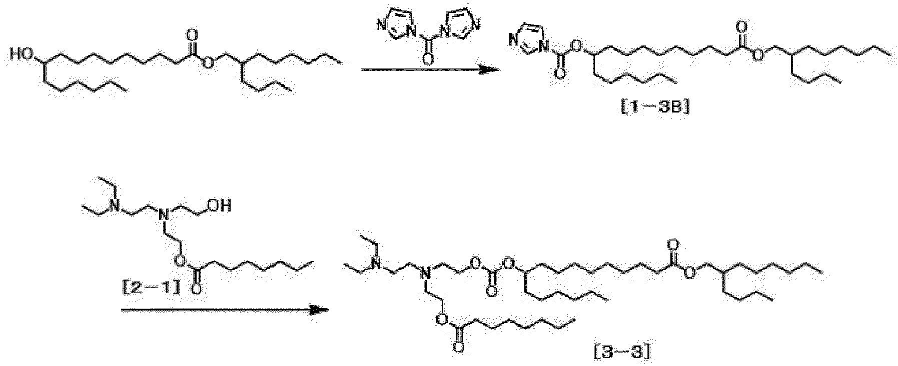

1,1'-Carbonyldiimidazole (6.6 g) was added to a tetrahydrofuran (60 mL) solution of 2-butyloctyl 10-hydroxyhexadecanoate (12.0 g), and the mixture was stirred at room temperature for 12 hours. Water (60 mL) and hexane (60 mL) were added to the reaction mixture, and the organic layer was separated and then washed with water (20 mL) and a saturated aqueous sodium chloride solution (20 mL). After drying over anhydrous sodium sulfate, the solvent was distilled off under reduced pressure to obtain a compound [1-3B] (16.7 g) as a light yellow oily substance.

¹H-NMR(CDCl₃) δ: 8.14-8.12 (1H, m), 7.43-7.41 (1H, m), 7.08-7.06 (1H, m), 5.11-5.04 (1H, m), 3.97 (2H, d, J = 5.6 Hz), 2.38-2.32 (2H, m), 1.80-1.54 (7H, m), 1.40-1.22 (34H, m), 0.91-0.85 (9H, m).

Acetonitrile (60 mL), the compound [2-1] (9.9 g), and potassium carbonate (7.9 g) were added to the obtained compound [1-3B] (16.7 g), and the mixture was stirred at 80°C for 2 hours. The reaction mixture was cooled to room temperature, then ethyl acetate (60 mL) and water (60 mL) were added thereto, the organic layer was separated, and the solvent was distilled off under reduced pressure. Ethyl acetate (60 mL), hexane (60 mL), water (60 mL), and methanol were added to the obtained residue, and the organic layer was separated. The organic layer was dried over anhydrous sodium sulfate, and then the solvent was distilled off under reduced pressure. The obtained residue was purified by silica gel column chromatography (methanol-ethyl acetate-hexane) to obtain a compound [3-3] (11.1 g, yield of 51% (two steps)) as a colorless oily substance.

¹H-NMR(CDCl₃) δ: 4.71-4.62 (1H, m), 4.20-4.08 (4H, m), 3.97 (2H, d, J = 5.6 Hz), 2.89-2.77 (4H, m), 2.73-2.42 (8H, m), 2.29 (4H, t, J = 7.6 Hz), 1.68-1.48 (9H, m), 1.39-1.18 (42H, m), 1.10-0.98 (6H, m), 0.94-0.81 (12H, m).

MS m/z (M + H): 798.

### [Comparative Example 4]

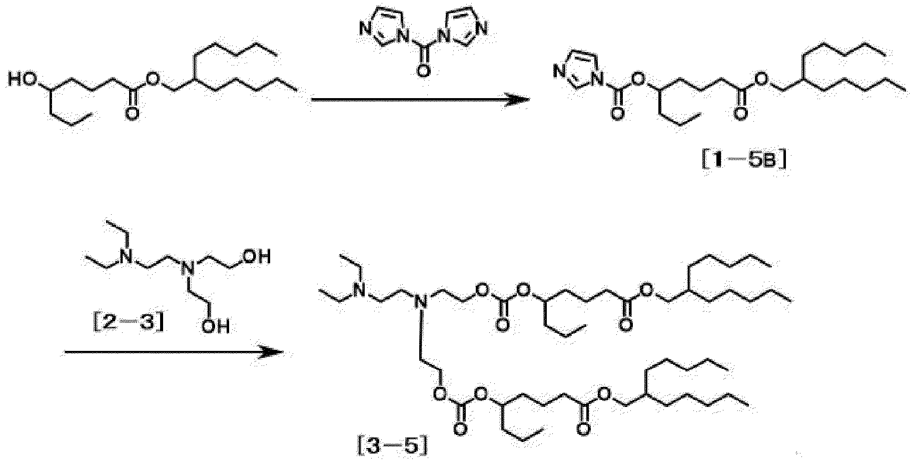

1,1'-Carbonyldiimidazole (296 mg) was added to a tetrahydrofuran (2 mL) solution of 2-pentylheptyl 5-hydroxyoctanoate (400 mg), and the mixture was stirred at room temperature for 1 hour. Water (4 mL), hexane (9 mL), and ethyl acetate (1 mL) were added to the reaction mixture, and the organic layer was separated, and then washed with a saturated aqueous sodium chloride solution. After drying over anhydrous sodium sulfate, the solvent was distilled off under reduced pressure to obtain a compound [1-5B] (505 mg) as a light yellow oily substance.

¹H-NMR(CDCl₃) δ: 8.14-8.12 (1H, m), 7.43-7.41 (1H, m), 7.08-7.06 (1H, m), 5.11-5.04 (1H, m), 3.97 (2H, d, J = 5.6 Hz), 2.38-2.32 (2H, m), 1.80-1.54 (7H, m), 1.40-1.22 (18H, m), 0.91-0.85 (9H, m).

Acetonitrile (2.5 mL), the compound [2-3] (122 mg), and potassium carbonate (165 mg) were added to the obtained compound [1-5B] (505 mg), and the mixture was stirred at 80°C for 2 hours. The reaction mixture was cooled to room temperature, then ethyl acetate (8 mL) and water (4 mL) were added thereto, and the organic layer was separated. The organic layer was washed with a saturated aqueous sodium chloride solution, and dried over anhydrous sodium sulfate, and the solvent was distilled off under reduced pressure. The obtained residue was purified by silica gel column chromatography (methanol-ethyl acetate-hexane) to obtain a compound [3-5] (304 mg, yield of 56%) as a colorless oily substance.

¹H-NMR(CDCl₃) δ: 4.74-4.67 (2H, m), 4.22-4.10 (4H, m), 3.97 (4H, d, J = 5.6 Hz), 2.83 (4H, t, J = 6.4 Hz), 2.69-2.65 (2H, m), 2.55-2.49 (6H, m), 2.34-2.30 (4H, m), 1.74-1.48 (14H, m), 1.43-1.20 (36H, m), 1.01 (6H, t, J = 7.2 Hz), 0.93-0.87 (18H, m).

MS m/z (M + H): 914.

### [Comparative Example 5]

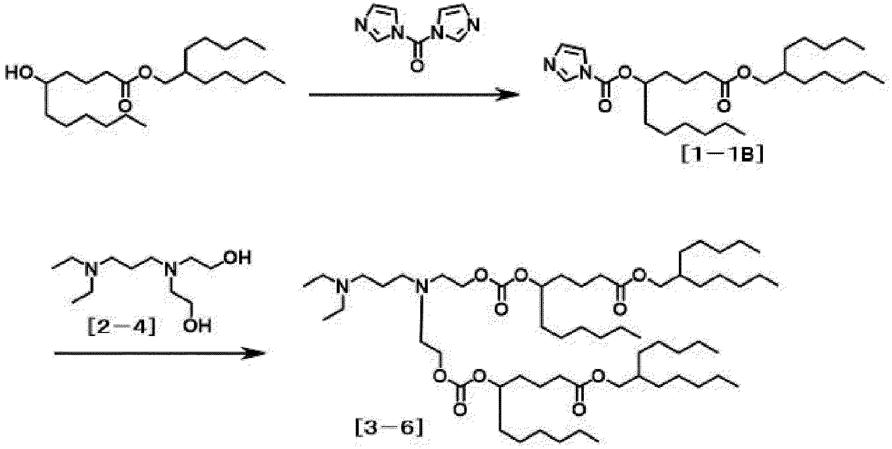

1,1'-Carbonyldiimidazole (0.53 g) was added to a tetrahydrofuran (4 mL) solution of 2-pentylheptyl 5-hydroxyundecanoate (0.80 g), and the mixture was stirred at room temperature for 1 hour. Water (4 mL) and hexane (4 mL) were added to the reaction mixture, and the organic layer was separated and then washed with a saturated aqueous sodium chloride solution. After drying over anhydrous sodium sulfate, the solvent was distilled off under reduced pressure to obtain a compound [1-1B] (1.18 g) as a colorless oily substance.

¹H-NMR(CDCl₃) δ: 8.14-8.12 (1H, m), 7.43-7.41 (1H, m), 7.08-7.06 (1H, m), 5.11-5.04 (1H, m), 3.97 (2H, d, J = 5.6 Hz), 2.38-2.32 (2H, m), 1.80-1.54 (7H, m), 1.40-1.22 (24H, m), 0.91-0.85 (9H, m).

Acetonitrile (2.5 mL), the compound [2-4] (0.12 g), and potassium carbonate (0.33 g) were added to the obtained compound [1-1B] (0.59 g), and the mixture was stirred at 80°C for 2 hours. The reaction mixture was cooled to room temperature, then ethyl acetate (3 mL) and water (3 mL) were added thereto, the organic layer was separated, and the solvent was distilled off under reduced pressure. Ethyl acetate (3 mL), hexane (3 mL), water (3 mL), and methanol were added to the obtained residue, and the organic layer was separated. The organic layer was washed with water and a saturated aqueous sodium chloride solution, and dried over anhydrous sodium sulfate, and the solvent was distilled off under reduced pressure. The obtained residue was purified by silica gel column chromatography (methanol-ethyl acetate-hexane) to obtain a compound [3-6] (0.26 g, yield of 47%) as a colorless oily substance.

¹H-NMR(CDCl₃) δ: 4.72-4.65 (2H, m), 4.22-4.09 (4H, m), 3.97 (4H, d, J = 6.0 Hz), 2.80 (4H, t, J = 6.4 Hz), 2.61-2.45 (6H, m), 2.44-2.37 (2H, m), 2.32 (4H, t, J = 7.2 Hz), 1.74-1.49 (16H, m), 1.35-1.21 (48H, m), 1.00 (6H, t, J = 7.2 Hz), 0.91-0.85 (18H, m).

MS m/z (M + H): 1012.

### [Comparative Example 6]

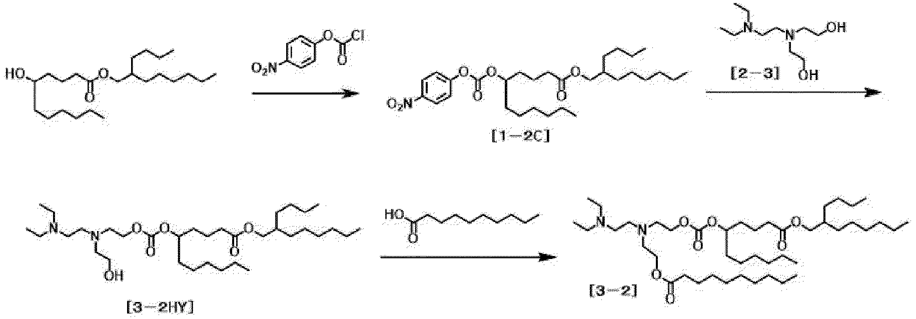

4-Nitrophenyl chloroformate (1.7 g) was added to a mixture of 2-butyloctyl 5-hydroxyundecanoate (1.6 g), triethylamine (2.4 mL), and tetrahydrofuran (16 mL), and the mixture was stirred at room temperature for 4 hours. Water and ethyl acetate were added to the reaction mixture, and the organic layer was separated. The organic layer was washed with water and dried over anhydrous sodium sulfate, and then the solvent was distilled off under reduced pressure. The obtained residue was purified by silica gel column chromatography (ethyl acetate-hexane) to obtain a compound [1-2C] (2.0 g) as a colorless oily substance.

¹H-NMR(CDCl₃) δ: 8.28 (2H, d, J = 9.3 Hz), 7.39 (2H, d, J = 9.3 Hz), 4.88-4.77 (1H, m), 3.99 (2H, d, J = 6.0 Hz), 2.41-2.31 (2H, m), 1.80-1.48 (7H, m), 1.44-1.20 (24H, m), 0.92-0.83 (9H, m).

Tetrahydrofuran (20 mL), the compound [2-3] (2.3 g), triethylamine (1.6 mL), and 4-dimethylaminopyridine (1.4 g) were added to the obtained compound [1-2C] (2.0 g), and the mixture was stirred at 80°C for 6 hours. The reaction mixture was cooled to room temperature, water and ethyl acetate were added thereto, and the organic layer was separated. The organic layer was washed with a saturated aqueous sodium chloride solution, and dried over anhydrous sodium sulfate, and the solvent was distilled off under reduced pressure. The obtained residue was purified by silica gel column chromatography (methanol-ethyl acetate) and silica gel column chromatography (NH silica gel, ethyl acetate-hexane) to obtain a compound [3-2HY] (1.1 g) as a colorless oily substance.

¹H-NMR(CDCl₃) δ: 4.75-4.64 (1H, m), 4.25-4.15 (2H, m), 3.97 (2H, d, J = 6.0 Hz), 3.54 (2H, t, J = 5.4 Hz), 2.89 (2H, t, J = 6.6 Hz), 2.75-2.63 (4H, m), 2.60-2.42 (6H, m), 2.33 (2H, t, J = 6.6 Hz), 1.73-1.50 (7H, m), 1.39-1.20 (24H, m), 1.03 (6H, t, J = 7.2 Hz), 0.95-0.81 (9H, m).

Dichloromethane (5 mL), decanoic acid (109 mg), triethylamine (0.35 mL), 4-dimethylaminopyridine (154 mg), and 1-ethyl-3-(3-dimethylaminopropyl)carbodiimide hydrochloride (161 mg) were added to the obtained compound [3-2HY] (250 mg), and the mixture was stirred at room temperature for 6 hours. Water and ethyl acetate were added to the reaction mixture, and the organic layer was separated and washed with water. After drying over anhydrous sodium sulfate, the solvent was distilled off under reduced pressure, and the obtained residue was purified by silica gel column chromatography (methanol-ethyl acetate) and silica gel column chromatography (NH silica gel, ethyl acetate-hexane) to obtain a compound [3-2] (195 mg, yield of 27% (3 steps))as a colorless oily substance.

¹H-NMR(CDCl₃) δ: 4.72-4.65 (1H, m), 4.22-4.09 (4H, m), 3.97 (2H, d, J = 5.6 Hz), 2.86-2.77 (4H, m), 2.70-2.64 (2H, m), 2.55-2.48 (6H, m), 2.35-2.27 (4H, m), 1.71-1.55 (9H, m), 1.36-1.20 (36H, m), 1.02 (6H, t, J = 7.2 Hz), 0.94-0.82 (12H, m).

MS m/z (M + H): 755.

### [Comparative Example 7]

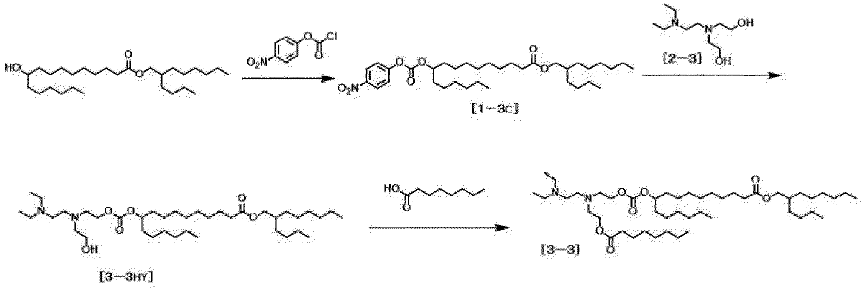

4-Nitrophenyl chloroformate (1.0 g) was added to a mixture of 2-butyloctyl 10-hydroxyhexadecanoate (1.5 g), triethylamine (1.4 mL), and tetrahydrofuran (15 mL), and the mixture was stirred at room temperature for 8 hours. Water and ethyl acetate were added to the reaction mixture, and the organic layer was separated. The organic layer was washed with water and then dried over anhydrous sodium sulfate, and the solvent was distilled off under reduced pressure. The obtained residue was purified by silica gel column chromatography (ethyl acetate-hexane) to obtain a compound [1-3C] (2.1 g) as a colorless oily substance.

¹H-NMR(CDCl₃) δ: 8.28 (2H, dd, J = 7.2 Hz, 2.1 Hz), 7.39 (2H, dd, J = 7.2 Hz, 2.1 Hz), 4.86-4.76 (1H, m), 3.97 (2H, d, J = 5.7 Hz), 2.30 (2H, t, J = 7.2 Hz), 1.74-1.20 (41H, m), 0.92-0.85 (9H, m).

Tetrahydrofuran (17 mL), the compound [2-3] (1.7 g), triethylamine (1.2 mL), and 4-dimethylaminopyridine (1.0 g) were added to the obtained compound [1-3C] (1.7 g), and the mixture was stirred at 80°C for 7 hours. The reaction mixture was cooled to room temperature, water and ethyl acetate were added thereto, and the organic layer was separated. The organic layer was washed with a saturated aqueous sodium chloride solution, and dried over anhydrous sodium sulfate, and the solvent was distilled off under reduced pressure. The obtained residue was purified by silica gel column chromatography (methanol-ethyl acetate) and silica gel column chromatography (NH silica gel, ethyl acetate-hexane) to obtain a compound [3-3HY] (1.2 g) as a colorless oily substance.

¹H-NMR(CDCl₃) δ: 4.75-4.61 (1H, m), 4.21 (2H, t, J = 6.6 Hz), 3.97 (2H, d, J = 5.7 Hz), 3.55 (2H, t, J = 5.1 Hz), 2.89 (2H, t, J = 6.6 Hz), 2.76-2.65 (4H, m), 2.64-2.41 (6H, m), 2.30 (2H, t, J = 8.1 Hz), 1.72-1.45 (7H, m), 1.40-1.20 (34H, m), 1.13-0.98 (6H, m), 0.96-0.81 (9H, m).

Dichloromethane (5 mL), octanoic acid (81 mg), triethylamine (0.31 mL), 4-dimethylaminopyridine (136 mg), and 1-ethyl-3-(3-dimethylaminopropyl)carbodiimide hydrochloride (142 mg) were added to the obtained compound [3-3HY] (250 mg), and the mixture was stirred at room temperature for 6 hours. Water and ethyl acetate were added to the reaction mixture, and the organic layer was separated and washed with water. After drying over anhydrous sodium sulfate, the solvent was distilled off under reduced pressure, and the obtained residue was purified by silica gel column chromatography (methanol-ethyl acetate) and silica gel column chromatography (NH silica gel, ethyl acetate-hexane) to obtain a compound [3-3] (271 mg, yield of 50% (3 steps))as a colorless oily substance.

¹H-NMR(CDCl₃) δ: 4.71-4.62 (1H, m), 4.20-4.08 (4H, m), 3.97 (2H, d, J = 5.6 Hz), 2.89-2.77 (4H, m), 2.73-2.42 (8H, m), 2.29 (4H, t, J = 7.6 Hz), 1.68-1.48 (9H, m), 1.39-1.18 (42H, m), 1.10-0.98 (6H, m), 0.94-0.81 (12H, m).

MS m/z (M + H): 798.

From the results of Examples and Comparative Examples, the yields of the target compounds in the manufacturing method according to the embodiment of the present invention (synthesis method A, Examples 1 to 6), the manufacturing method described in WO2021/095876A (synthesis method B, Comparative Examples 1 to 5), and the manufacturing method described in WO2019/235635A (synthesis method C, Comparative Examples 6 and 7) are summarized in Table 1.

**[Table 1]**

| Each synthesis method and yield | | | |
|---|---|---|---|
| Target compound | Yield (%) | | |
| | Synthesis method A | Synthesis method B | Synthesis method C |
| [3-1] | 83 | 65 | - |
| [3-2] | 85 | 66 | 26 |
| [3-3] | 77 | 51 | 50 |
| [3-4] | 79 | - | - |
| [3-5] | 73 | 56 | - |
| [3-6] | 88 | 47 | - |

From the above results, it was clarified that the target compound can be synthesized with a higher yield than the conventional method by using the manufacturing method according to the embodiment of the present invention.

The cause of the improvement in yield of the present invention was considered. Under the conditions of Comparative Example 1, it was considered that the imidazole that was eliminated from the compound [1-1B] because of the progress of the reaction promoted the disproportionation reaction of the compound [2-1] to generate the compound [2-3] and a compound [2-3DA], and a plurality of by-products such as a compound [3-1HY] and a compound [3-1DA] were generated through these compounds, which resulted in a low yield. On the other hand, under the conditions of Example 1, it was considered that since the reactivity of the triazole released from the compound [1-1] is lower than that of imidazole, the disproportionation reaction of the compound [2-1] is extremely slow, and the generation of by-products is suppressed, which resulted in a high yield.

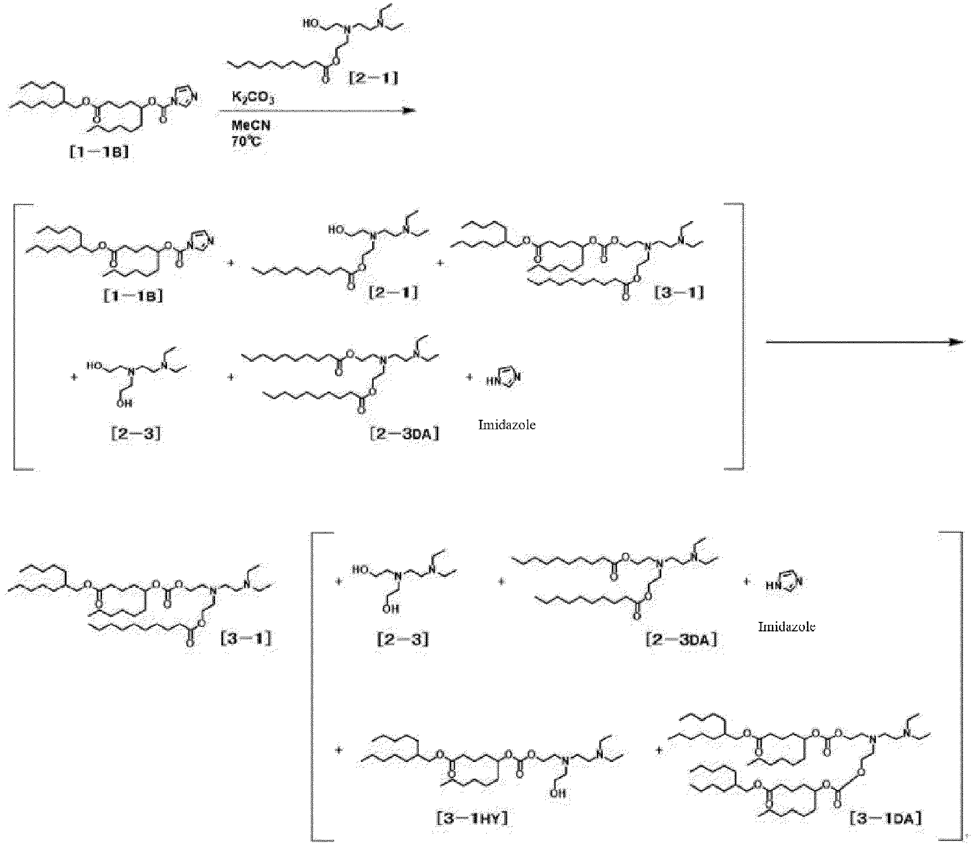

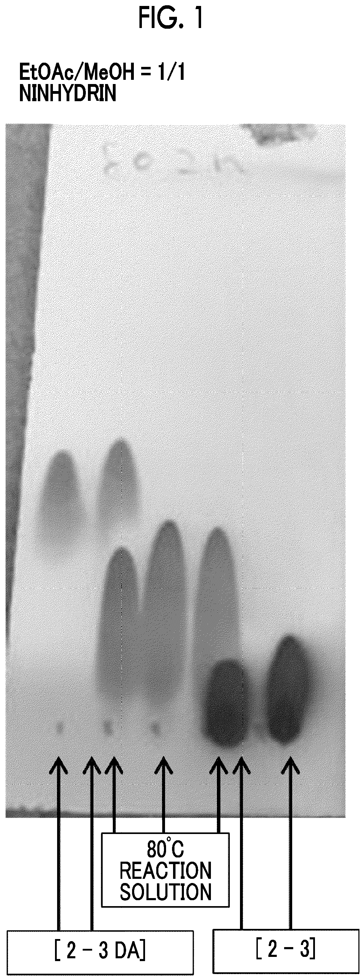

The compound [2-1] in the reaction solution under the reaction conditions of Comparative Example 1 was analyzed by silica gel thin layer chromatography. Potassium carbonate (25 mg) was added to a mixture of the compound [2-1] (100 mg) and acetonitrile (0.5 mL), and the mixture was stirred at 80°C for 2 hours and analyzed by silica gel thin layer chromatography (developing solvent: ethyl acetate/methanol of 1/1, color developing reagent: ninhydrin). The results are shown in Fig. 1. It was found that the compound [2-1] did not react under the conditions of Comparative Example 1.

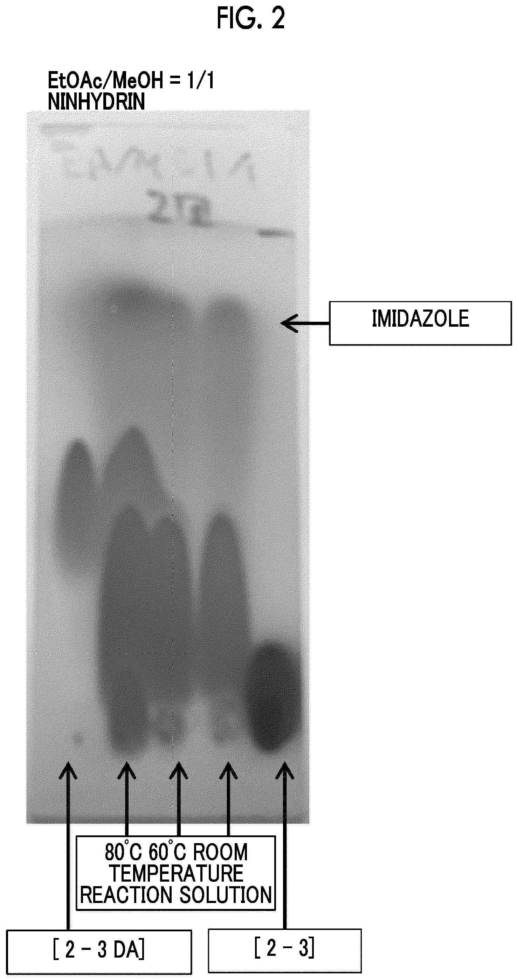

In a case where imidazole was added under the reaction conditions of Comparative Example 1, the compound [2-1] in the reaction solution was analyzed by silica gel thin layer chromatography. Imidazole (20 mg) was added to a mixture of the compound [2-1] (100 mg), potassium carbonate (25 mg), and acetonitrile (0.5 mL), and the mixture was stirred at room temperature, 60°C, or 80°C for 3 hours, and then subjected to silica gel thin layer chromatography analysis (developing solvent: ethyl acetate/methanol of 1/1, color developing reagent: ninhydrin). The results are shown in Fig. 2. It was found that the compound [2-1] was converted into the compound [2-3] and the compound [2-3DA] at a reaction temperature of 60°C or 80°C under the condition in which imidazole was added under the condition of Comparative Example 1.

From the above, it is considered that, under the conditions of Comparative Example 1, the disproportionation reaction of the compound [2-1] is promoted by the increase in the imidazole generated with the progress of the reaction, and the compound [2-3] and the compound [2-3DA] are generated, and a plurality of by-products are generated through these, which results in a decrease in a yield.

## Claims

1. A compound represented by Formula [1] or a salt thereof, 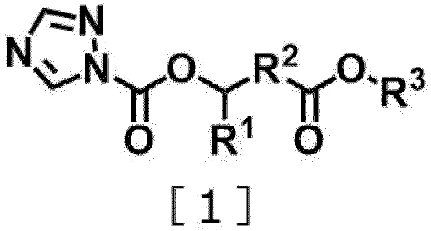 In the formula,
R¹ represents a hydrogen atom or a hydrocarbon group having 1 to 12 carbon atoms,
R² represents a divalent hydrocarbon group having 2 to 12 carbon atoms, and
R³ represents a hydrocarbon group having 6 to 22 carbon atoms.

2. The compound or a salt thereof according to claim 1,
wherein R¹ represents a hydrocarbon group having 3 to 6 carbon atoms.

3. The compound or a salt thereof according to claim 1 or 2,
wherein R² represents a divalent hydrocarbon group having 3 to 8 carbon atoms.

4. The compound or a salt thereof according to claim 1 or 2,
wherein R³ represents a hydrocarbon group having 10 to 14 carbon atoms.

5. The compound or a salt thereof according to claim 1 or 2,
wherein R³ represents a group represented by -R³¹-C(R³²)R³³, where R³¹ represents a divalent hydrocarbon group having 1 to 5 carbon atoms and R³² and R³³ each independently represent a hydrocarbon group having 1 to 8 carbon atoms.

6. A compound represented by Formula [1-1], Formula [1-2], Formula [1-3], Formula [1-4], Formula [1-5], Formula [1-6], Formula [1-7], Formula [1-8], or Formula [1-9], or a salt thereof,

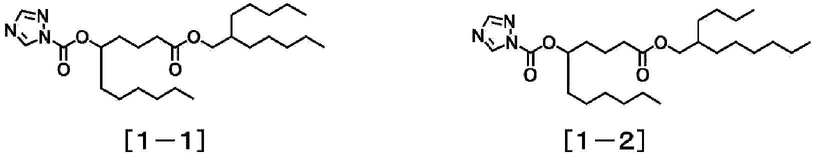

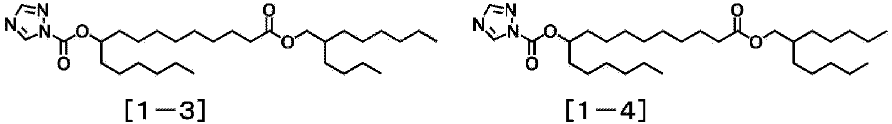

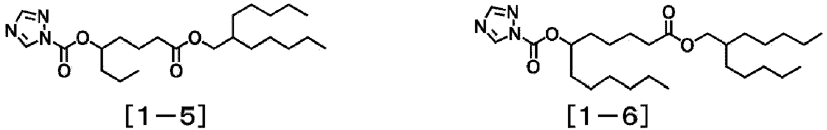

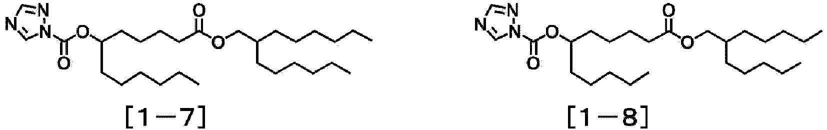

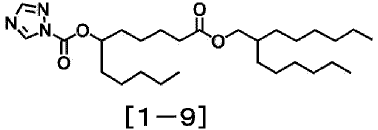

7. A method for manufacturing a compound represented by Formula [3] or a salt thereof, the method comprising:
reacting the compound represented by Formula [1] or a salt thereof according to claim 1 with a compound represented by Formula [2] or a salt thereof, 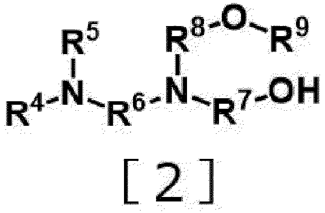 in the formula,
R⁴ and R⁵ each independently represent a hydrocarbon group having 1 to 3 carbon atoms,
R⁶ represents a divalent hydrocarbon group having 2 to 4 carbon atoms,
R⁷ and R⁸ each independently represent a divalent hydrocarbon group having 2 to 4 carbon atoms, and
R⁹ represents a hydrogen atom or -COR¹⁰, where R¹⁰ represents a hydrocarbon group having 5 to 18 carbon atoms, 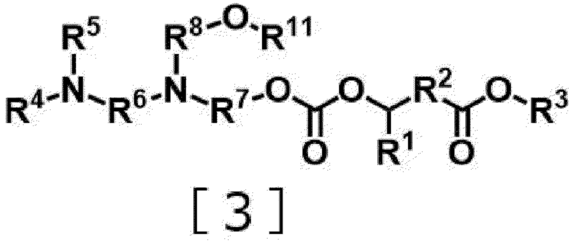 in the formula,
R¹, R², and R³ have the same definition as R¹, R², and R³ in Formula [1],
R⁴, R⁵, R⁶, R⁷, and R⁸ have the same definition as R⁴, R⁵, R⁶, R⁷, and R⁸ in Formula [2], and
R¹¹ represents -COR¹⁰ or 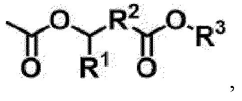 where R¹⁰ has the same definition as R¹⁰ in Formula [2], and R¹, R², and R³ have the same definition as R¹, R², and R³ in Formula [1].

8. The method according to claim 7,
wherein R⁴ and R⁵ represent a hydrocarbon group having 2 carbon atoms.

9. The method according to claim 7 or 8,
wherein R⁶ represents a divalent hydrocarbon group having 2 or 3 carbon atoms.

10. The method according to claim 7 or 8,
wherein R⁷ and R⁸ each independently represent a divalent hydrocarbon group having 2 carbon atoms.

11. The method according to claim 7 or 8,
wherein R⁹ represents -COR¹⁰, where R¹⁰ represents a hydrocarbon group having 7 to 9 carbon atoms.

12. The method according to claim 7 or 8,
wherein any one of the following (a), (b), (c), (d), (e), (f), (g), (h), (i), or (j) is applied,
(a) the compound represented by Formula [1] or a salt thereof is a compound represented by Formula [1-1] or a salt thereof, the compound represented by Formula [2] or a salt thereof is a compound represented by Formula [2-1] or a salt thereof, and the compound represented by Formula [3] or a salt thereof is a compound represented by Formula [3-1] or a salt thereof,
(b) the compound represented by Formula [1] or a salt thereof is a compound represented by Formula [1-2] or a salt thereof, the compound represented by Formula [2] or a salt thereof is a compound represented by Formula [2-1] or a salt thereof, and the compound represented by Formula [3] or a salt thereof is a compound represented by Formula [3-2] or a salt thereof,
(c) the compound represented by Formula [1] or a salt thereof is a compound represented by Formula [1-3] or a salt thereof, the compound represented by Formula [2] or a salt thereof is a compound represented by Formula [2-2] or a salt thereof, and the compound represented by Formula [3] or a salt thereof is a compound represented by Formula [3-3] or a salt thereof,
(d) the compound represented by Formula [1] or a salt thereof is a compound represented by Formula [1-4] or a salt thereof, the compound represented by Formula [2] or a salt thereof is a compound represented by Formula [2-2] or a salt thereof, and the compound represented by Formula [3] or a salt thereof is a compound represented by Formula [3-4] or a salt thereof,
(e) the compound represented by Formula [1] or a salt thereof is a compound represented by Formula [1-5] or a salt thereof, the compound represented by Formula [2] or a salt thereof is a compound represented by Formula [2-3] or a salt thereof, and the compound represented by Formula [3] or a salt thereof is a compound represented by Formula [3-5] or a salt thereof,
(f) the compound represented by Formula [1] or a salt thereof is a compound represented by Formula [1-1] or a salt thereof, the compound represented by Formula [2] or a salt thereof is a compound represented by Formula [2-4] or a salt thereof, and the compound represented by Formula [3] or a salt thereof is a compound represented by Formula [3-6] or a salt thereof,
(g) the compound represented by Formula [1] or a salt thereof is a compound represented by Formula [1-6] or a salt thereof, the compound represented by Formula [2] or a salt thereof is a compound represented by Formula [2-4] or a salt thereof, and the compound represented by Formula [3] or a salt thereof is a compound represented by Formula [3-7] or a salt thereof,
(h) the compound represented by Formula [1] or a salt thereof is a compound represented by Formula [1-7] or a salt thereof, the compound represented by Formula [2] or a salt thereof is a compound represented by Formula [2-4] or a salt thereof, and the compound represented by Formula [3] or a salt thereof is a compound represented by Formula [3-8] or a salt thereof,
(i) the compound represented by Formula [1] or a salt thereof is a compound represented by Formula [1-8] or a salt thereof, the compound represented by Formula [2] or a salt thereof is a compound represented by Formula [2-4] or a salt thereof, and the compound represented by Formula [3] or a salt thereof is a compound represented by Formula [3-9] or a salt thereof, or
(j) the compound represented by Formula [1] or a salt thereof is a compound represented by Formula [1-9] or a salt thereof, the compound represented by Formula [2] or a salt thereof is a compound represented by Formula [2-4] or a salt thereof, and the compound represented by Formula [3] or a salt thereof is a compound represented by Formula [3-10] or a salt thereof,
